(19) Europäisches Patentamt
European Patent Office
Office européen des brevets

(11) EP 1 049 678 B1

(12) **EUROPÄISCHE PATENTSCHRIFT**

(45) Veröffentlichungstag und Bekanntmachung des Hinweises auf die Patenterteilung:
**24.10.2012 Patentblatt 2012/43**

(21) Anmeldenummer: **98947540.5**

(22) Anmeldetag: **21.09.1998**

(51) Int Cl.:
***C12Q 1/68*** *(2006.01)*

(86) Internationale Anmeldenummer:
**PCT/EP1998/006002**

(87) Internationale Veröffentlichungsnummer:
**WO 1999/015509 (01.04.1999 Gazette 1999/13)**

(54) **CYCLOHEXYL- UND HETEROCYCLYL-NUKLEOSID DERIVATE, DIE HERSTELLUNG UND VERWENDUNG DIESER DERIVATE, DEREN OLIGOMERE BZW. KONJUGATE IN PAARUNGS- UND/ODER TESTSYSTEMEN**

CYCLOHEXYL AND HETEROCYCLYL NUCLEOSIDE DERIVATIVES, METHOD FOR PRODUCING THESE DERIVATIVES, AND THE USE OF THE DERIVATIVES AND THEIR OLIGOMERS OR CONJUGATES IN PAIRING AND/OR TESTING SYSTEMS

DERIVES DE NUCLEOSIDE DE CYCLOHEXYLE ET D'HETEROCYCLYLE, LEUR PREPARATION ET LEUR UTILISATION, LEURS OLIGOMERES ET CONJUGUES DANS DES SYSTEMES D'APPARIEMENT ET/OU DE TEST

(84) Benannte Vertragsstaaten:
**AT CH DE FR GB IT LI NL**

(30) Priorität: **22.09.1997 DE 19741739**

(43) Veröffentlichungstag der Anmeldung:
**08.11.2000 Patentblatt 2000/45**

(73) Patentinhaber: **SANOFI**
**75008 Paris (FR)**

(72) Erfinder:
- **MICULKA, Christian**
  **D-65929 Frankfurt am Main (DE)**
- **WINDHAB, Norbert**
  **D-65759 Hattersheim (DE)**
- **ESCHENMOSER, Albert**
  **CH-8700 Küsnacht (CH)**
- **SCHERER, Stefan**
  **D-64572 Büttelborn (DE)**
- **QUINKERT, Gerhard**
  **D-61479 Glashütten (DE)**

(74) Vertreter: **Ackermann, Joachim et al**
**Postfach 11 13 26**
**60048 Frankfurt am Main (DE)**

(56) Entgegenhaltungen:
**WO-A-93/13121 WO-A-97/43232**
**WO-A-98/25943**

- **SCHAEFFER H.J. & VINCE R.: "Enzyme inhibitors. XIX. The synthesis of some 1-hydroxy-2-hydroxymethyl-4-(6-substituted -9-purinyl) cyclohexanes as nucleoside analogs" JOURNAL OF MEDICINAL CHEMISTRY, Bd. 11, Nr. 1, Januar 1968, Seiten 15-20, XP002090866**
- **PEREZ-PEREZ M.J. ET AL.: "Application of the Mitsunobu-type condensation reaction to the synthesis of phosphonate derivatives of cyclohexenyl and cyclohexanyl nucleosides" JOURNAL OF ORGANIC CHEMISTRY, Bd. 60, Nr. 6, Juni 1995, Seiten 1531-1537, XP002090867**
- **MIKHAILOV S.N. ET AL.: "Use of cyclohexene epoxides in the preparation of carbocyclic nucleosides" NUCLEOSIDES & NUCLEOTIDES, Bd. 15, Nr. 4, 1996, Seiten 867-878, XP002089763**
- **MARQUEZ V.E. & LIM M.I.: "Carbocyclic nucleosides" MEDICINAL RESEARCH REVIEWS, Bd. 6, Nr. 1, 1986, Seiten 1-40, XP000607502**
- **BORTHWICK A.D. & BIGGADIKE K.: "Synthesis of chiral carbocyclic nucleosides" TETRAHEDRON (REPORT NUMBER 305), Bd. 48, Nr. 4, 24. Januar 1992, Seiten 571-623, XP000563919**
- **KNAPP S.: "Iodolactamization: Aspects and applications" ADVANCES IN HETEROCYCLIC NATURAL PRODUCT SYNTHESIS, Bd. 3, 1996, Seiten 57-98, XP002090868**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 049 678 B1

## Beschreibung

Die vorliegende Erfindung betrifft eine Verbindung der Formel I

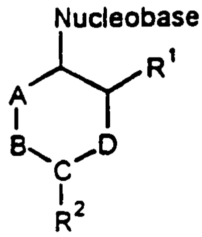

(I),

ihre Herstellung und Verwendung in Paarungs- und/oder Testsystemen

Paarungssysteme sind supramolekulare Systeme nicht kovalenter Wechselwirkung, die sich durch Selektivität, Stabilität und Reversiblität auszeichnen, und deren Eigenschaften bevorzugt thermodynamisch, d. h. durch Temperatur, pH-Wert und Konzentration beeinflußt werden. DNA und RNA spielen dabei als Träger der Erbanlagen eine fundamentale Rolle. Solche Paarungssysteme können z. B. aufgrund ihrer selektiven Eigenschaften aber auch als "molekularer Klebstoff" für die Zusammenführung von unterschiedlichen Metallelustern zu Cluster-Verbänden mit potentiell neuen Eigenschaften verwendet werden [R. L. Letsinger, et al., Nature, 1996, 382, 607-9; P. G. Schultz et al., Nature, 1996, 382, 609-11).

Starke und thermodynamisch kontrollierbare Paarungssysteme spielen daher eine immer wichtigere Rolle für die Anwendung im Bereich der Nanotechnologie, zur Herstellung neuer Materialien, Diagnostika, Therapeutika sowie mikroelektronischer, photonischer und optoelektronischer Bauteile und für das kontrollierte Zusammenführen molekularer Species zu supramolekularen Einheiten, wie z. B. den (kombinatorischen) Aufbau von Proteinassemblies [siehe z. B. A. Lombardi, J. W. Bryson, W. F. DeGrado, Biomoleküls (Pept. Sci.) 1997, 40, 495-504].

Zur Herstellung derartiger Paarungssysteme besitzen DNA- bzw. RNA-Bausteine jedoch folgende Nachteile:

a) Die Kräfte, die zwei Stränge zusammenhalten, vor allem Wasserstoffbrücken und Stapeleffekte, sind naturgemäß sehr gering. Solche Duplices weisen daher eine geringe Stabilität auf Dies kann durch Aufnahme einer sog. Umwandlungskurve und Ermittlung des Umwandlungspunktes leicht festgestellt werden. Folglich sind für die Herstellung von Paarungssysteme relativ lange Einzelstränge notwendig, was zur Folge hat, daß der Anteil des Paarungssystems an der supramolekularen Einheit überwiegt, d. h. die "Nucleotidlast" ist hoch.

b) Durch die Ausbildung von Hoogsteen-Paarungen, die alternativ zu *Watson-Crick*-Paarungen möglich sind, nimmt die Selektivität ab. Damit sind oftmals parallele Duplices oder irreversible Paarungsvorgänge verbunden.

c) Durch die hohe Flexibilität des Zucker-Phosphat-Rückgrates bilden sich helicale Konformationen, wodurch die räumliche Anordnung in supramolekularen Einheiten weniger gut gesteuert werden kann.

d) Die chemische Instabilität der Phosphodiesterbindung im Rückgrat läßt nur eine geringe Varianz an physikalischen Bedingungen, wie pH oder Temperatur, für die Verwendung der supramolekularen Einheiten zu.

e) Die Nuclease-Empfindlichkeit der Produkte führt zu einem nur schwer vermeidbaren, raschen enzymatischen Abbau und damit zur Zerstörung der supramolekularen Einheit.

f) Eine mögliche Interferenz mit dem genetischen Material biologischer Systeme ist nicht auszuschließen, falls die supramolekularen Einheiten in einem biologischen System zum Einsatz kommen, d. h. eine Orthogonalität des Paarungsvorganges fehlt.

g) Die Herstellung größerer Mengen an Oligonucleotiden ist aufgrund der geringen Beladbarkeit der üblicherweise verwendeten Festphase beispielsweise im Vergleich zur Peptidsynthese schwierig.

h) Die Herstellung der nicht-natürlichen L-enantiomeren Form ist durch die schlechte Zugänelichkeit der entsprechend konfigurierten Zuckerbausteine erschwert.

Damit ist eine Verwendung von DNA- bzw. RNA-Bausteinen z. B. in komplementär gebundenen zwei- und dreidimensionalen supramolekularen Strukturen (siehe z. B. WO96/13522) in einem physiologischen Medium vor allem im Hinblick auf die Punkte e), f) und g) nur schwer zu realisieren.

Eine Alternative zu DNA- bzw. RNA-Bausteinen ist die sogenannte Pyranosyl-RNA (p-RNA). pRNA ist ein Oligonucleotid, das anstelle der Ribofuranose der RNA die Ribopyranose als Zuckerbaustein enthält und daher ausschließlich *Watson-Crick*-gepaarte, antiparallele, reversibel "schrnelzende, quasi-lineare und stabile Duplices ausbildet.

Daneben gibt es auch homochirale p-RNA-Stränge entgegengesetzten Chiralitätssinns, die ebenfalls kontrollierbar paaren und in der gebildeten Duplex nicht streng-helical sind. Diese für den Aufbau supramolekularer Einheilen wertvolle Sperifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich letzfich auf die Teilnahme eines 2',4'-cisdisubstituierten Ribopyranoserings am Aufbau des Rückgrates zurückführen. p-RNA löst damit einige der beschriebenen Nachteile von DNA und RNA, jedoch nicht die Nachteile gemäß Punkt d), e), g) und h).

Weitere carbocyclische Nucleoside werden in den Dokumenten WO 93/13121 A; WO 97/43232 A; Schneffer H.J. et al., Journal of Medicinal Chemistry, Bd. 11, Nr. 1, Januar 1968, 15-20; Perez-Perez MJ. et al., Journal of Organic Chemistry, Bd. 60, Nr. 6, Juni .1995, 1531-1531; Mikhailov S:N., et al., Nucleosides & Nucleotides, Bd. 15, Nr. 4, 1996, 867-878; Marquez V.E. et al., Medicinal Research Reviews, Bd 6, Nr. 1, 1986, 1-40 und Borthwick AD. et al., Tetrahedron, Bd. 48, Nr. 4, 1992, 571-623. Kapp S., Advances in Heterocyclic Natural Product Synthesis, Bd. 3, 1996, 57-98 beschäftigt sich mit verschiedenen Aspekten und Anwendungen der Iodlactamisierung.

Eine weitere Alternative stellt die Verknüpfung der Monomereinheiten über Amidbindungen, d. h. der Aufbau eines oligomeren Peptids, sogenannten Peptide Nucleic Acids (PNA's), dar.

PNA's weisen durch ihre offenkettige Struktur eine hohe Flexibilität auf und sind damit hinsichtlich ihrer konformationellen Präorganisation für den kontrollierten Aufbau supramolekularer Systeme nicht geeignet.

Aufgabe der vorliegenden Erfindung ist es daher, Verbindungen bereit zu stellen, die die oben beschriebenen Nachteile nicht aufweisen.

Ein Gegenstand der vorliegenden Erfindung sind daher Verbindungen der Formel I

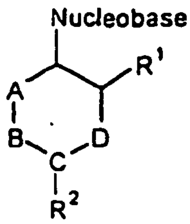

(I)

worin $R^1$ gleich $NR^3R^4$, $OR^3$, oder $SR^3$ ist mit $R^3$ und $R^4$ unabhängig voneinander, gleich oder verschieden, H oder $C_nH_{2n+1}$, wobei n eine ganze Zahl von 1-12, vorzugsweise 1-8, insbesondere 1-4 ist; vorzugsweise $R^1$ gleich $NR^3R^4$ oder $OR^3$, insbesondere $NR^3R^4$, vor allem $NH_2$ bedeutet;

$R^2$ gleich $C_mH_{2m}$-C(X)-Y ist mit X gleich =O oder =S ; Y gleich $OR^3$, $NR^3R^4$ oder $SR^3$, wobei $R^3$ und $R^4$ die oben genannte Bedeurung haben; und m eine ganze Zahl von 1-4, insbesondere 1-3, vor allem 1-2; und Y ist vorzugsweise gleich $OR^3$ oder $NR^3R^4$; oder

$R^2$ gleich $C_mH_{2m}$-Z-Y' ist mit Z gleich eine Sulfonyl-, Phosphonyl-, Ether- oder Amin-Gruppe, wobei, wenn Z gleich eine Sulfonyl- oder Phosphonyl-Gruppe, Y' gleich H, $C_nH_{2n+1}$, $OR^3$, $NR^3R^4$ oder $SR^3$ ist, wobei n, $R^3$ und $R^4$ die oben genannte Bedeutung haben, und, wenn Z gleich eine Ether- oder Amin-Gruppe, Y' gleich $C_nH_{2n+1}$, ist;

A, B, und D unabhängig voneinander, gleich oder verschieden, $CR^5R^6$ bedeuten mit $R^5$, $R^6$ unabhängig voneinander H oder $C_nH_{2n+1}$, wobei n die oben genannte Bedeutung hat; und C gleich $CR^8$ bedeutet, mit $R^3$ das unabhängig davon die Bedeutung von $R^5$ besitzt; und der Begiff Nucleobase im Sinne der vorliegenden Erfindung Thymin, Uracil, Adenin, Cytosin, Guanin, Isocytosin, Isoguanin, Xanthin oder Hypoxanthin, vorzugsweise Thymin, Uracil, Adenin, Cytosin oder Guanin bedeutet, wobei die Verbindungen der Formel I die Verbindungen 1-[2-(tert.-Butoxycarbonyl)-amino-4-(carboxymethyl)cyclohexyl]thymin, 1-[2-(tert-Butoxycarbonyl)-amino-4-(carboxymethyl)cyclohexyl]-3-[(Benryloxy)methyl]-thymin und 1-[2-(tert-Butoxycarbonyl)-amino-4-(carboxymethyl)cyclohexyl]-6-N-p-methoxybenzoyl-adenin nicht umfassen.

Insbesondere sind Verbindungen bevorzugt, bei denen $R^1$ gleich $NH_2$ und $R^2$ gleich $CH_2$-COOH ist, vor allem eine [2-Amino-4-(carboxymethyl)cyclohexyl]-Nucleobase, wie 1-[2-Amino-4-(carboxymethyl)cyclohexyl]-thymin, 1-[2-Amino-4-(carboxymethyl)cyclo-hexyl]-uracil, 1-[2-Amino-4-(carboxymethyl)cyclohexyl]-cytosin, 9-[2-Amino-4-(carboxymethyl)-cyclohexyl]-adenin oder 9-[2-Amino-4-(carboxymethyl)cyclohexyl]-guanin.

Weiterhin ist es vorteilhaft, wenn die erfindungsgemäße Verbindung enantiomerenrein ist.

Für die Synthese ist es ferner vorteilhaft, wenn $R^1$ auch mit Schutzgruppen versehen ist, wie z. B. im Falle von $R^1$ gleich $NH_2$ mit tert.-Butoxycarbonyl-Gruppen (BOC) oder 9-Fluorenylmethoxycarbonyl-Gruppen (FMOC) oder im Falle von $R^1$ gleich OH mit Ether- oder Acetalschutzgruppen. Unter Schutzgruppen versteht man im allgemeinen Reste, die reaktive Gruppen von Verbindungen vor unerwünschten Reaktionen schützen und leicht abspaltbar sind. Derartige

Gruppen sind z. B. Benzyloximethyl- (BOM-), BOC-, FMOC-, Ether- oder Acetalschutzgruppen.

Daneben kann Y auch mit Aktivierungsreagentien zu reaktiven Intermediaten, z. B. gemischten Anhydriden, umgesetzt sein.

Erfindungsgemäße Verbindungen sind Cyclohexanderivate, die in 1'-Stellung die Nucleobase und in 2'-Stellung ein Nucleophil, wie z. B. ein Stickstoffatom tragen, das mit der reaktiven Gruppe in 4'-Stellung bzw. nach deren Aktivierung, wie z. B. miteiner Carbonylfunktion in Reaktion gebracht werden kann, und somit durch Wiederholung dieses Vorgangs in der Lage ist, eine oligomere Struktur aufzubauen. Aus stereochemischer Sicht sind Derivate bevorzugt, bei denen alle Substituenten am Sechsring äquatoriale Positionen aufweisen, insbesondere bei denen die Substituenten in 1'- und 2'-Stellung äquatoriale Postionen aufweisen und vor allem bei denen die Nucleobase eine äquatoriale Position aufweist.

Die Verbindungen der Formel I können beispielsweise folgendermaßen hergestellt werden.

Am Anfang wird eine chirogene Diels-Alder-Reaktion zwischen z. B. 1,3-Butadien **1** und z. B. 3-(2-Propenoyl)-1,3-oxazolidin-2-on **2** in Gegenwart chiral, nicht racemischer Promotoren wie das Seebach'sche TADDOL **3** (D. Seebach et al., J. Org. Chem. 1995, 60, 1788) durchgeführt (siehe Fig. 1). 3-(2-Propenoyl)-1,3-oxazolidin-2-on kann beispielsweise durch Umsetzen von Oxazolidin-2-on mit Acrylsäurechlorid in Gegenwart von Kupfer und Kupfer(I)chlorid hergestellt werden. Die Verbindung 5 kann beispielsweise aus **4** in Anwesenheit von Magnesiumspänen und Wasser-freiem Methanol hergestellt werden. Das Reaktionsprodukt wird anschließend z. B. mit Lithiumaluminiumhydrid reduziert, um die Verbindung **6** zu ergeben. Das Acetoniril **7** kann aus **6** z. B. durch Umsetzen mit Methansäurechlorid zum Methansäuremethylester und anschließend durch Umsetzen mit Cyanid hergestellt werden. Eine alkalische Hydrolyse des Reaktionsproduktes ergibt das Essigsäurederivat, welches mit $SOCl_2$ zum Acetylchlorid umgesetzt werden kann, um in Anwesenheit von einer wäßrigen Ammoniaklösung das Acetamid **8** zu ergeben. Anschließend erfolgt dessen Jodlactamisierung (S. Knapp et al., Org. Synth. 1991, 70, 101), zum Jodlactam **9.**

Anschließend kann das Jodlactam **9** in Anwesenheit eines Hydrids an eine Nucleobase gekoppelt werden, ohne daß der Bicyclus zerstört wird.

Hierzu ist es vorteilhaft, wenn eventuell reaktive Gruppen der Nucleobase durch geeignete Schutzgruppen, z. B. BOC, FMOC, Acetal usw. geschützt werden. Lediglich beim Diaminopurin als Nucleobase sind Schutzgruppen nicht nötig. Jedoch hat sich gezeigt, daß bei Diaminopurin als Nucleobase der Lactamring nicht mehr geöffnet werden konnte. Unabhängig davon spielt Diaminopurin in biologischen Systemen keine Rolle.

Weiterhin ist es bevorzugt, wenn das Jodlactam nicht in der racemischen Form, sondern enantiomerenrein vorliegt, da die aufzubauenden Oligomeren, die ein Paarungssystem darstellen sollen, nur einer sterochemischen Reihe angehören sollen. Der Aufbau eines Paarungssystems aus racemischen Bausteinen ist im allgemeinen nicht möglich.

Gemäß der vorliegenden Erfindung kann ein kopplungsfähiger Pyrimidinbaustein, z. B. ein Thyminbaustein, ausgehend vom Iodlactam **9** in einer fünfstufigen Synthesequenz in einer Ausbeute von 27 % über alle Schritte hergestellt werden (Fig. 2.):

> Im ersten Schritt erfolgt die stereoselektive Einführung der Nucleobase in einer Substitutionsreaktion (**13a**) gefolgt von der Maskierung des aciden Imidprotons des Thymins (**13b**)**.** Anschließend wird das Lactam durch Einführen einer Schutzgruppe, z. B. der Boc-Gruppe (**13c**), aktiviert und der Ring nucleophil z. B. mittels LiOOH (**14a**) geöffnet. Nach Entfernen der beispielsweise vorhandenen BOM-Schutzgruppe ergibt sich mittels katalytischer Hydrierung der gewünschte Baustein (**14b**).

Der enantiomorphe (R)-Baustein ent-**14b** ist durch analoges Vorgehen aus dem (R)-Iodlactam ent-**9** erhältlich. Ebenso sind in analoger Weise kopplungsfähige Uracil-, Cytosin- und Isocytosin-Bausteine erhältlich.

Ein kopplungsfähiger Purinbaustein, z. B. ein Adeninbaustein, ist ausgehend vom Iodlactam 9 in einer siebenstufigen Synthesequenz in einer Ausbeute von 19 % über alle Schritte zugänglich (Fig. 3.):

> Nach Einführung des z. B. N(6)-benzoylierten Adenins in das (S)-Iodlactam **9** unter Retention (**15a**) erfolgt die Umschützung zum z. B. N(6)-Formamidinadeninlactam **15e.** Anschließend erfolgt die Aktivierung des Lactams z. B. mittels tert-Butoxycarbonylierung (**15d**), ein erneutes Umschützen zum z. B. N(6)-anisoylgeschützten Lactam (**151**) und nachfolgende Lactamspaltung z. B. mittels LiOH liefert den gewünschten Baustein **16.**

Der enantiomorphe (R)-Baustein **ent-16** ist durch analoges Vorgehen aus dem (R)-Iodlactam ent-9 erhältlich. Ebenso sind in analoger Weise kopplungsfähige Guanin-, Isoguanin-, Xanthin-, und Hypoxanthin-Bausteine erhaltlich.

Die Herstellung von Heteroatom-enthaltenden Sechsringen sowie anderen Derivaten der erfindungsgemäßen Verbindung sind z. B. analog über eine Hetero-Diels-Alder-Reaktion bzw. über Diels-Alder-Reaktionen mit den entsprechenden Ausgangsverbindungen möglich.

Ein anderer Gegenstand der vorliegenden Erfindung ist daher ein Verfahren zur Herstellung einer erfindungsgemäßen Verbindung mit folgenden Schritten:

(a) Kopplung des entsprechenden Jodlactams, vorzugswiese eines enantiomerenreinen Jodlactams mit einer geschützten Nücleobase, vorzugsweise in Anwesenheit eines Hdrids, wie z. B. NaH,
(b) Aktivierung des Lactams, beispielsweise durch Einführen einer Schutzgruppe wie z. B. eine BOC-Gruppe, sowie
(c) nucleophile Ringöffnung, z. B. durch ein Hydroperoxid wie z. B. LiOOH.

Zur Herstellung von Oligomeren werden die erfindungsgemäßen Verbindungen dann vorzugsweise an fester Phase nach Art der Merrifield-Peptidsynthese zu oligomeren Strukturen aufgebaut. Dabei werden die notwendigen Reagenzien vorzugsweise im Überschuß zugesetzt und nicht umgesetzte Mengen durch einfache Waschschritte wieder entfernt [R. B. Merrifield, J. Amer. Chem. Soc. 1963, 85, 2149]. Repetitive Zyklen, die aus Abspaltungen der temporären Schutzgruppen und Kupplungen, z. B. nach der Methode des symmetrischen Anhydrids, der geschützten Monomer-Bausteine bestehen, führen zum Oligomeren am Harz. Am Ende der Synthese wird die terminale Schutzgruppe abgespalten, das Oligomer vom Harz gespalten und bevorzugt mit chromatographischen Methoden aufgereinigt. Die isolierten HPLC-Fraktionen der Syntheseprodukte wurden mit analytischer HPLC auf ihre Reinheit überprüft und mit Elektrospray-Massenspektrometrie eindeutig charakterisiert.

Ein weiterer Gegenstand der vorliegenden Erfindung ist daher ein Oligomer gemäß Anspruch 16, CNA (Cyclohexylnucleooligoamid) genannt, enthaltend mindestens eine erfindungsgemäße Verbindung, welche 2'->4' verknüpft sind.

Um molekulare Species, wie Peptide, Proteine, Rezeptoren, Redoxzentren, Antikörper, Nucleinsäure-Abschnitte (wie DNA, RNA) mit Hilfe des beschriebenen Paarungssystem zusammenzuführen, werden im allgemeinen geeignete Linker integriert. Bevorzugt, aber nicht ausschließlich, läßt sich Lysin an jeder beliebigen Stelle im oligomeren Paarungssystem, ganz bevorzugt aber terminal, einbauen. Lysin hat durch seine freie Aminogruppe eine Vielzahl von Verknüpfungsmöglichkeiten. Der Einbau in das erfindungsgemäße Oligomer erfolgt beispielsweise über Boc-Lysin (Fmoc), dessen FMOC-Gruppe mit Piperidin in DMF abgespalten werden kann.

Daher bezieht sich die vorliegende Erfindung auch auf Oligomere, die zusätzlich mindestens einen Linker, vorzugsweise einen Lysinlinker, vor allem einen terminalen Lysinlinker enthalten.

Das beispielsweise aminofunktionalisierte Oligomere kann auch bevorzugt mit aktivierten Linkern wie z. B. Jodacetylsuccinimid (A) oder Bis(hydroxysuccinimidyl)glutarat (B) derivatisiert werden, welche dann bevorzugt in physiologischen Medien, insbesondere in wäßrig-gepufferten Lösungen ggf. unter Zugabe von organischen Lösungsvermittlern, mit HS-Gruppen der molekularen Spezie, z. B. über (A) mit den Cystein-Resten eines Peptids oder Proteins oder über (B) mit Aminogruppen der molekularen Spezie unter Bildung von chemisch stabilen, kovalenten Bindungen reagieren.

Daher umfaßt die vorliegende Erfindung auch Oligomere, die mit einem aktivierten Linker, wie z. B. Jodacetylsuccinimid und/oder Bis(hydroxysuccinimidyl)glutarat derivatisiert sind.

Die erfindungsgemäßen Oligomere mit freiem N- und C-Terminus sind bei pH 7 im allgemeinen nur schlecht in Wasser löslich. Bei Tetrameren werden Konzentrationen im 100 $\mu$molaren, bei Pentameren im 10 $\mu$molaren und bei Hexameren nur noch etwa im 1 $\mu$molaren Bereich erreicht. Dies sind Richtwerte, die je nach Sequenz auch um eine Größenordnung über oder unterschritten werden können. Die Löslichkeit für AAATT liegt beispielsweise bei 1$\mu$M, die für AATAT bei 50 $\mu$M.

Durch Acylierung des N-Terminus mit einem Hydroxycarbonsäurederivat läßt sich beispielsweise eine Hydroxyfunktion einführen, die phosphoryliert werden kann. Das so erhaltene phosphatierte Oligomere weist bei ca. pH 7 in Wasser üblicherweise eine mindestens ca. 1000-fach erhöhte Löslichkeit auf Der Nachweis der Löslichkeit erfolgt im allgemeinen durch UV-Spektroskopie. Die vorliegende Erfindung bezieht sich daher auch auf phosphatierte Oligomere.

Ein anderer Gegenstand der Erfindung ist auch ein Konjugat aus einem erfindungsgemäßen Oligomer und einem Biomolekül.

Unter Biomolekül versteht man gemäß der vorliegenden Erfindung z. B. ein Peptid, Peptoid oder Protein, wie z. B. einen Rezeptor, einen Antikörper oder funktionelle Teile davon oder ein Enzym, sowie eine Nucleinsäure wie DNA oder RNA, oder Zellbestandteile wie Lipide, Glykoproteine, Filamentbestandtelle, oder Viren, Virenbestandteile wie Kapside, Viroide, und deren Derivate wie z. B. Acetate.

Funktionelle Teile von Antikörper sind beispielsweise Fv-Fragmente (Skerra & Plückthun (1988) Science 240, 1038), einzelkettige Fv-Fragmente (scFv; Bird et al (1988), Science 242, 423; Huston et al. (1988) Proc. Natl. Acad. Sci. U.S.A., 85, 5879) oder Fab-Fragmente (Better et al. (1988) Science 240, 1041). Die erfindungsgemäßen Konjugate aus Effektormolekülen und bevorzugt peptidischen, aber im Gegensatz zu PNA selektiven und kontrollierbaren Paarungssystemen sind dann vorteilhaft, wenn reversibel supramolekulare *assemblies* aufgebaut werden sollen, deren Wirkung, wie z. B. Bindung, Inhibition, Auslösung eines physiologischen Effektes, sich von der Wirkung der einzelnen Effektormoleküle unterscheidet.

So beschreibt z. B. WO 96/13613 eine Methode zum Auffinden einer Substanz, die eine biologische Wirkung durch die Multimerisierung von Proteinen auslöst, indem man zuerst eine Substanz I durch einen Test ermittelt, die an ein Protein bindet, dann eine zweite Substanz II ermittelt, die an ein zweites Protein bindet und dann die beiden Substanzen I und II kovalent über einen Linker verbindet, so daß eine Dimerisierung der beiden Proteine ausgelöst wird. Diese Dimerisierung führt dann den gewünschten biologischen Effekt herbei. Größere Flexibilität kann eine solche Methode erhalten, wenn das Verbinden der beiden Substanzen I und II nicht kovalent, sondern durch ein Paarungssystem wie das erfindungsgemäße Oligomer oder Konjugat erfolgt. Durch die Steuerbarkeit dieser Paarung, beispielsweise durch Temperatur oder pH, kann der Dimerisierungsvorgang der Proteine beobachtet oder dessen Ausmaß gesteuert werden. Die erfindungsgemäßen Paarungssysteme haben z. B. gegenüber den Systemen aus WO 96/13522 den Vorteil, daß sie Nuclease-stabil sind.

Ein Ansatz, peptidische "Klebe"-Einheiten zur Bildung von homo- oder heterodimeren *assemblies* zu verwenden, wird z. B. in WO 94/28173 beschrieben:

Assoziierungspeptide (Hexa- oder Heptapeptide) einer festgelegten Sequenz führen Effektoreinheiten, wie z. B. Proteine, zu supramolekularen Einheiten zusammen. Höhere Flexibilität kann eine solchen Methode durch Steuerbarkeit dieses Assoziierungsvorganges erhalten, die im allgemeinen mit den Assoziierungspeptiden nicht realisiert werden kann, in vorteilhafter Weise jedoch mit den Paarungssystemen der vorliegenden Erfindung.

Daher bezieht sich die Erfindung auch auf die Verwendung der erfindungsgemäßen Verbindung, des erfindungsgemäßen Oligomers bzw. des erfindungsgemäßen Konjugates in einem Paarungs- und/oder Testsystem, wie z. B. in WO94/28173, WO96/135 22, WO96/13613, R. L. Letsinger, et al., Nature, 1996, 382, 607-9; P. G. Schultz et al., Nature, 1996, 382, 609-11 oder A. Lombardi, J. W. Bryson, W. F. DeGrado, Biomoleküls (Pept. Sci.) 1997, 40, 495-504 näher beschrieben und oben allgemein erläutert.

Eine besondere Ausführungsform der vorliegenden Erfindung stellt ein Träger dar, an den eine erfindungsgemäße Verbindung, ein erfindungsgemäßes Oligomer und/oder ein erfindungsgemäßes Konjugat immobilisiert ist, insbesondere zur Verwendung in einem Paarungs- und/oder Testsystem, wie oben näher beschrieben.

Unter dem Begriff "immobilisiert" versteht man im Sinne der vorliegenden Erfindung die Ausbildung einer kovalenten Bindung, quasi-kovalenten Bindung oder supramolekularen Bindung durch Assoziation von zwei oder mehreren molekularen Spezien wie linear konstituierte Moleküle, insbesondere Peptide, Peptoide, Proteine, lineare Oligo- oder Polysaccharide, Nukleinsäuren und deren Analoga, oder Monomere wie Heterocyclen, insbesondere Stickstoffheterocyclen, oder nichtlinear konstituierte Moleküle wie verzweigte Oligo- oder Polysacharide oder Antikörper und deren funktionelle Teile wie Fv-Fragmente, einzelkettige Fv-Fragmente (scFv) oder Fab-Fragmente.

Als Trägermaterialien eigenen sich beispielsweise Keramik, Metall, insbesondere Edelmetall, Gläser, Kunststoffe, kristalline Materialien bzw. dünne Schichten des Trägers, insbesondere der genannten Materialien, oder (bio) molekulare Filamente wie Cellulose, Gerüstproteine.

Zusammenfassend weist die vorliegende Erfindung gegenüber herkömmlichen Paarungs- und Testsystemen folgende-Vorteile auf:

Die Duplices der erfindungsgemäßen Oligomere oder Konjugate sind wesentlich stabiler als jene der DNA, RNA und p-RNA. Die oligomeren Substanzen sind chemisch und gegenüber enzymatischem Abbau im wesentlichen stabil, paaren nicht mit DNA oder RNA und lassen sich auf einfache Weise in größeren Mengen herstellen. Beide Enantiomere sind durch synthetische chirogene Schritte leicht zugänglich. Die erfindungsgemäßen Verbindungen sind daher als selektive "molekulare Klebstoffe" den bisher bekannten Paarungssystemen überlegen.

Die folgenden Figuren und Beispiele sollen die Erfindung näher beschreiben, ohne sie zu beschränken.

BESCHREIBUNG DER FIGUREN

Fig. 1 zeigt schematisch die Synthese des Jodlactams 9.
Fig. 2 zeigt schematisch die Synthese eines kopplungsfähigen Thyminbausteins.
Fig. 3 zeigt schematisch die Synthese eines kopplungsfähigen Adeninbausteins.
Fig. 4 zeigt die Abbildung einer reversiblen UV-Umwandlungskurve des Oligomers ATATA.
Fig. 5 zeigt die Umwandlungskurve von phosphatiertem Hba-AATAT.

BEISPIELE

Beispiel 1

*Synthese eines kopplungsfähigen Thyminbausteins*

**Herstellung von 1-[(*S*, *S*, *S*)-8-Azabicyclo[3.3.1]nonan-7-on-2-yl]-3-[(benzyloxy)methyl]thymin (13b)**

**1. Durchführung**

In einem trocknen, mit Schutzgas begasten 50-ml-Löwenthalkolben wurden 148 mg (4.00 mmol) 65%ige NaH-Suspension vorgelegt, zweimal mit je 5 ml wasserfreiem Pentan gewaschen und in 20 ml DMF suspendiert. Es wurden zuerst portionsweise 493 mg (2.00 mmol) 3-(Benzyloxy)methylthymin und nach Abklingen der Gasentwicklung 530 mg (2.00 mmol) 9 zugesetzt. Nach ca. 20 minütigem Rühren bei Raumtemp. bildete sich eine fahlgelbe Reaktionslösung, die über Nacht bei Raumtemp. gerührt wurde.

Man versetzte mit 1 ml einer ges wäßrigen $NH_4Cl$-Lösung und überführte die Reaktionslösung unter Nachspülen mit MeOH in einen Rundkolben. Im Anschluß entfernte man die Lösungsmittel zunächst vorsichtig iV.i.RV, danach durch Kugelrohrdestillation (70°C/0.5 Torr). Man erhielt 1.71 g eines gelborangefarbenen Öles, welches durch MPL-Chromatographie an 150 g Kieselgel 60 ($CH_2Cl_2$/MeOH 30:1) gereinigt wurde. Der so erhaltene Feststoff wurde mit wenig $Et_2O$ digeriert, abfiltriert, zerkleinert und im ÖPV getrocknet. Dies lieferte 405 mg (53%) **13b** (*ent*-**13b**: 50%, *rac*-**13**: 51%) als farbloses Pulver vom Schmp. 160-162°C. Eine analytische Probe wurde durch Kristallisation aus THF/Cyclohexan erhalten.

**2. Analytische Daten**

*1-[*(S,S, S)*-8-Azabicyclo[3.3.1]nonan-7-on-2-yl]-3-[(benzyloxy)methyl]thymin* (**13b**):

| | |
|---|---|
| **Schmp.:** | 160-162°C (THF/Cyclohexan); *ent*-**13b** : 161-162°C. rac-13b: 186-188°C (THF/ Cyclohexan). |
| **DC:** | $CH_2Cl_2$/MeOH 15:1, $R_f$ 0.4 1. |
| **Spez. Drehung:** | $[\alpha]_{589}^{20} = -43.3°$ (c = 1.10, $CH_2Cl_2$); ent-**13b**: $[\alpha]_{589}^{20} = +42.9°$ (c = 1.05, $CH_2Cl_2$). |
| **UV** ($CH_3CN$): | $\lambda_{max}$ 274.2 (9623). |
| **IR** (KBr): | 3447*w*, 3189*w*, 3090*w*, 2949*m*, 2906*m*, 1703*s*, 1652*s*, 1464*s*, 1405*s*, 1357*s*, 1270*s*, 1076*s*. |
| **$^1$H-NMR** (300 MHz, $d_6$-DMSO): | 1.59 ($m_c$, 3H, H-C(4'), 2H-C(9')); 1.85 ($m_c$, H-C(3')), 1.88 (*s*, $CH_3$); 2.00-2.14 (*m*, 3H, H'-C(3'), H'-C(4'), H-C(6')); 2.20 (br. *s*, H-C(5')); 2.47 (*dd, J*(H'-C(6'), H-C(6')) = 18.5, *J*(H'-C(6'), H-C(5')) = 7.5, H'-C(6')); 3.58 (br. *m*, H-C(1')); 4.22 (br. *m*, H-C (2')); 4.60 (*s*, 2H, PhC$H_2$O), 5.35 ($m_c$ 2H, OC$H_2$N); 7.22-7.35 (*m*, 5H, Ph); 7.54 (s *m*. Fs., H-C(6)); 7.99 (d, *J*(H-N(8'), H-C(1')) = 4.6, H-N(8')). |

Die Zuordnung der Signale erfolgte mit Hilfe eines $^1$H,$^1$H-COSY-Spektrums.

| | |
|---|---|
| **$^{13}$C-NMR** (75 MHz, $d_6$-DMSO): | 12.75 ($CH_3$); 17.90 (C(3')); 23.82 (C(9'); 24.66 (C(5')); 27.47 (C(4')); 37.15 (C(6')); 47.27 (C(1')); 55.55 (C(2')); 70.45 ($OCH_2N$); 71.06 ($PhCH_2O$); 107.82 (C(5)); 127.06, 127.29, 128.03 (5C, Ph); 137.40 (C(6)); 138.18 ($C_{ipso}$); 151.04 (C(2)); 162.67 (C(4)); 170.83 (C(7')). |

Die Zuordnung der Signale erfolgte mit Hilfe eines $^1$H,$^{13}$C-COSY-Spektrums.

| | | |
|---|---|---|
| **MS** ($ESI^-$): | 767.8 [2M+H], 384.5 [M+H]. | |
| **Anal.:** | er. für $C_{21}H_{25}N_3O_4$ (383.45): | b C 65.78, H 6.57, N 10.96; |
| | gef.: | C 65.68, H 6.64, N 10.84. |

**Herstellung von 1-[(*S, S, S*)-8-Aza-8-(*tert*-butoxycarbonyl)-bicyclo[3.3.1]nonan-7-on-2-yl)-3-[(benzyloxy)methyl] thymin (13c)**

**1. Durchführung**

In einem 250-ml-Rundkolben wurden 7.18 g (18.7 mmol) **13b** in 100 ml $CH_2Cl_2$ gelöst und nacheinander mit 2.59 ml (18.7 mmol, leq.) $NEt_3$ und 8.00 ml (37.4 mmol, 2eq.) $Boc_2O$ versetzt. Im Anschluß gab man spatelweise 2.28 g (18.7 mmol, 1eq.) DMAP zu und rührte die gelbe Reaktionslösung vier Stunden bei Raumtemp.. Es wurden nochmals nacheinander 3.89 ml (28.1 mmol, 1.5 eq.) $NEt_3$, 12.0 ml (56.1 mmol, 3 eq.) $Boc_2O$ und 3.43 g (28.1 mmol, 1.5 eq.) DMAP zugegeben. Nach Rühren über Nacht unter Wasserausschluß waren laut DC-Kontrolle (Kieselgel 60; $CH_2Cl_2$/MeOH 15:1) immer noch Spuren Edukt vorhanden. Daher wurden im Abstand von vier Stunden nochmals je 2.00 ml (9.3 5 mmol) $Boc_2O$ zugesetzt. Man ließ erneut über Nacht unter Wasserausschluß rühren und entfernte im Anschluß die Lösungsmittel und Reagenzien i.V.i.RV. Dies lieferte 19.5 g eines schmierigen, gelben Feststoffes, den man an 275 g Kieselgel 60 (AcOEt/n-Heptan 2:1) chromatographierte. Man erhielt 8.50 g eines leicht gelb gefärbten Feststoffes. Umkristallisation aus 50 ml THF und 100 ml Cyclohexan lieferte 7.81 g (86%) **13c** (*ent*-**13c**: 87%; *rac*-**13c**: 85%) als farblose, kleine Kristalle vom Schmp. 124-126°C.

**2. Analytische Daten**

*1-[(*S,S, S*)-8-Aza- 8-(tert-butoxycarbonyl)bicyclo[3.3.1]nonan-7-on-2-yl)-3-[(benzyloxy)methyl]thymin* (**18**):

| | |
|---|---|
| **Schmp**.: | 124-126°C (THF/Cyclohexan); *ent*-**13c**: 123-125°C. *rac*-13c: 125-127°C (THF/Cyclohexan). |
| **DC:** | AcOEt/*n*-Heptan 2:1, $R_f$ 0.13. |
| **Spez. Drehung:** | $[\alpha]_{589}^{20} = -10.3°$ (c = 1.37, $CH_2Cl_2$); *ent*-**13c**: $[\alpha]_{589}^{20} = +10.6°$ (c = 1.20, $CH_2Cl_2$). |
| **UV** ($CH_3CN$): | $\lambda_{max}$ 273.6 (10200). |
| **IR (KBr):** | 3447*w*, 2934*w*, 1744*s*, 1701*m*, 1663*s*, 1448*m*, 1363*m*, 1260*s*, 1153*m*, 1078*m*. |
| **$^1$H-NMR** (300 MHz, $CDCl_3$): | 1.49 (s, 9H, $C(CH_3)_3$); 1.52-1.63, 1.74-2.13 (2*m*, 6H, 2H-C(3'), 2H-C(4'), 2H-C(9')); 1.88 (*s*, $CH_3$-C(5)); 2.33 (br. *s*, H-C(5')); 2.38 (*d*, *J*(H-C(6'), H'-C(6')) = 18.2, H-C(6')); 2.70 (*dd*, *J*(H'-C(6'), H-C(6')) = 18.2, *J*(H'-C(6'), H-C(5')) = 6.7, H'-C(6')); 4.27 ($m_c$, H-C(1')); 4.40 ($m_c$, H-C(2')); 4.65 (s, 2H, Ph$CH_2$O); 5.45 ($m_c$, 2H, O$CH_2$N); 7.14-7.32 (*m*, 6H, H-C(6), Ph). |

Die Zuordnung der Signale erfolgte mit Hilfe eines $^1$H,$^1$H-*COSY* Spektrums.

| | |
|---|---|
| **$^{13}$C-NMR** (50 MHz, $CDCl_3$): | 13.39 ($CH_3$-C(5)), 19.63 (C(3')); 24.90 (C(9'); 25.37 (C(5')); 27.39 (C(4')); 27.84 (C($CH_3$)$_3$); 41.03 (C(6')); 53.36 (C(1')); 57.71 (C(2')); 70.80 (O$CH_2$N); 72.25 (Ph$CH_2$O); 89.97 ($C(CH_3)_3$); 109.73 (C(5)); 127.55, 128.20 (5C, Ph); 137.17 (C(6)); 138.16 ($C_{ipso}$); 151.19, 152.15 ($O_2CN$, C(2)); 163.30 (C(4)); 170.45 (C(7')). |

Die Zuordnung der Signale erfolgte mit Hilfe eines $^1$H, $^{13}$C-*COSY*-Spektrums.

| | | |
|---|---|---|
| **MS** (ESI$^+$): | 501.7 [M+H], 384.5 [M+H-Boc]. | |
| **Anal.:** | ber. für $C_{26}H_{33}N_3O_6$ (483.56): | C 64.58, H 6.88, N 8.69; |
| | gef.: | C 64.45, H 6.91, N 8.59. |

**Herstellung von 3-[(Benzyloxy)methyl)-1-[(*S*,*S*,*S*)-2-(*tert*-butoxycarbonyl)amino-4-(carboxymethyl)cyclohexyl] thymin (14a)**

**1. Durchführung**

In einem 500-ml-Rundkolben wurden 7.81 g (16.2 mmol) **13c** in 225 ml THF gelöst, mit 50 ml Wasser versetzt und mittels Eisbad auf 0°C abgekühlt. Es wurden 7.33 g (64.8 mmol) 30%iges Wasserstoffperoxid gefolgt von 25 ml einer wäßrigen Lösung von 1.36 g (32.3 mmol) LiOH-Monohydrat zugegeben, woraufhin sich die Lösung trübte. Das Eisbad wurde entfernt und der Reaktionsansatz 45 min gerührt. Die Reaktionslösung wurde mit 25 ml einer 1.5M wäßrigen $Na_2SO_3$-Lösung und 75 ml ges. $NaHCO_3$-Lösung versetzt. Im Anschluß wurde das THF i.V.i.RV weitgehend entfernt. Die Lösung wurde auf 350 ml Wasser gegossen und mit wenig 2N Natronlauge auf pH > 12 eingestellt. Die milchige Suspension wurde dreimal mit je 350 ml $CH_2Cl_2$ extrahiert und die ver. org. Phasen mit $MgSO_4$ getrocknet. Nach Entfernen der Lösungsmittel i. V.i.RV erhielt man 2.00 g verunreinigtes **13b**, das aus 30 ml THF und 60 ml Cyclohexan umkristallisiert wurde. Es gelang so 1.55 g (25%) **13b**(*ent*-**13b**: 27%; *rac*-**13b**: 30%) wiederzugewinnen.

Die basische, wäßrige Phase wurde vorsichtg mit halbkonzentrierter Salzsäure auf pH 1-2 eingestellt und dreimal mit je 350 ml AcOEt extrahiert. Nach Trocknen mit $Na_2SO_4$, Entfernen der Lösungsmittel 1.V.i.RV, digerieren mit 50 ml $Et_2O$, Filtration und Trocknen im ÖPV erhielt man 5.09 g (63%) **14a** (*ent*-**14a**: 60%; *rac*-**14a**: 65%) als feinkristallinen Feststoff vom Schmp. 89-91°C.

**2. Analytische Daten**

*3-[(Benzyloxy)methyl]-1-[(*S,S,S*)-2-(*tert*-butoxycarbonyl)amino-4-(carboxymethyl)-cyclohexyl]thymin* (**14a**):

| | |
|---|---|
| **Schmp**.: | 89-91°C (nach Digerieren mit $Et_2O$); *ent*-**14a**: 90-92°C. *rac*-**14a**: 168-170°C (THF/ Cyclohexan). |
| **DC**: | $CH_2Cl_2$/MeOH 15:1, $R_f$ 0.37. |
| **Spez. Drehung**: | $[\alpha]_{589}^{20} = -24.0°$ (c = 0.99, MeOH); *ent*-14a: $[\alpha]_{589}^{20} = +23.9°$ (c = 1.05, MeOH). |
| **UV** ($CH_3CN$): | $\lambda_{max}$ 273.2 (10890). |
| **IR** (KBr): | 3373*m*, 2974*m*, 2932*m*, 1706*s*, 1691*s*, 1664*s*, 1647*s*, 1534*m*, 1469*m*, 1452*m*, 1365*m*, 1290*m*, 1255*m*, 1175*m*. |
| **$^1$H-NMR** (300 MHz, $d_6$-DMSO): | 0.88-1.30 (*m*, 2H, H-C(4) H-C(6)); 1.19 (*s*, 9H, $C(CH_3)_3$), 1.66-2.00 (*m*, 5H, 2H-C (3), H'-C(4), H-C(5), H'-C(6)); 1.80 (*s*, $CH_3$-C(5')), 2.16 ($m_c$, 2H, $CH_2COOH$); 3.79 ($m_c$, H-C(1)); 4.27 ($m_c$, H-C(2)); 4.58 (*s*, 2H, $PhCH_2O$); 5.34 (*s*, 2H, $OCH_2N$); 6.86 (*d*, *J*(H-N, H-C(1)) = 9.6, NH); 7.23-7.37 (m, 5H, Ph); 7.68 (br. *s*, H-C(6')); 12.09 (br. *s*, $CH_2COOH$). |

Die Zuordnung der Signale erfolgte mit Hilfe eines $^1$H, $^1$H-*COSY*-Spektrums.

| | |
|---|---|
| **$^{13}$C-NMR** (50 MHz, $CDCl_3$): | 13.09 ($CH_3$-C(5')); 28.11 ($C(CH_3)_3$); 30.13 (C(3)); 31.07 (C(4); 33.38 (C(5)); 39.23 (C (6)); 40.18 ($CH_2COOH$); 51.38 (C(1)); 58.19 (C(2)); 70.83 ($OCH_2N$); 72.17 ($PhCH_2O$); 80.05 ($C(CH_3)_3$); 110.06 (C(5')); 127.62, 127.69, 128.27 (5C, Ph); 135.46 (C(6')); 138.00 ($C_{ipso}$); 152.72, 155.43 ($O_2CN$, C(2')); 163.20 (C(4')); 176.30 ($CH_2COOH$). |

Die Zuordnung der Signale erfolgte mit Hilfe eines $^1$H, $^{13}$C-*COSY*-Spektrums.

| | | |
|---|---|---|
| **MS** ($ESI^-$): | 519.7 [M+H+$H_2O$). | |
| **Anal**.: | ber. für $C_{26}H_{35}N_3O_7$ (501.58): | C 62.26, H 7.03, N 8.38; |
| | gef.: | C 62.00, H 6.96, N 8.17. |

**Herstellung von 1-[(*S*,*S*,*S*)-2-(*tert*-Butoxycarbonyl)amino-4-(carboxymethyl)cyclohexyl]thymin (14b)**

**1. Durchführung**

In einem 500-ml-Dreihalskolben wurden 300 mg Pd-C in 60 ml wasserfreiem THF suspendiert und 45 min lang mit Wasserstoff gesättigt. Es wurde im Anschluß eine Lösung von 2.01 g (4.00 mmol) **14a** in 30 ml THF zugegeben und die Reaktionsmischung 90 min (DC-Kontrolle; $CH_2Cl_2$/MeOH 15:1) intensiv unter Wasserstoffatmosphäre gerührt.

Der Katalysator wurde über *Celite* abfiltriert und die Lösungsmittel i.V.i.RV entfernt. Man erhielt 1.96 g eines farblosen Schaumes, den man in 90 ml wasserfreiem MeOH löste und mit 454 mg (8.40 mmol) NaOMe versetzte. Es wurde über Nacht unter Wasserausschluß bei Raumtemp. gerührt.

Man versetzte mit 6 ml ges. $NH_4Cl$-Lösung und entfernte die Lösungsmittel weitgehend i.V.i.RV. Der Rückstand wurde auf 50 ml Wasser gegossen, der pH-Wert der wäßrigen Phase mit 2N Salzsäure auf 1-2 eingestellt und die wäßrige Phase viermal mit je 50 ml AcOEt extrahiert. Nach Trocknen mit $Na_2SO_4$ und Entfernen der Lösungsmittel i.V.i.RV digerierte man den erhaltenen Schaum mit 20 ml $Et_2O$. Nach Filtration des Präzipitates und Trocknen im ÖPV erhält man 1.43 g (94%) **14b**(*ent*-**14b**: 90%; *rac*-**14b**: 88%) als farblosen Feststoff vom Schmp. 231-233°C. Eine analytische Probe wurde durch Kristallisation aus MeOH/Wasser erhalten.

**2. Analytische Daten**

*1-[(*S,S,S)*-2-(*tert*-Butoxycarbonyl)amino)-4-(carboxymethyl)cyclohexyl]thymin* (**14b**):

| | |
|---|---|
| **Schmp.**: | 235-237°C (MeOH/ $H_2O$); Gasentwicklung; *ent*-**14b**: 234-237°C. *rac*-**14b**: 231-233°C (MeOH/ $H_2O$); Gasentwicklung.u. Zersetz.. |
| **DC**: | $CH_2Cl_2$/MeOH 5:1, $R_f$ 0.38. |
| **Spez. Drehung**: | $[\alpha]_{589}^{20} = -21.9°$ (c = 0.57, MeOH); *ent*-**14b**: $[\alpha]_{589}^{20} = +21.2°$ (c = 0.53, MeOH). |
| **UV** ($H_2O$): | $\lambda_{max}$ 272.4 (11450). |
| **IR** (KBr): | 3374*s*, 3187*w*, 2978*w*, 2935*w*, 1702*s*, 1648*s*, 1522*s*, 1394*w*, 1366*w*, 1282*s*, 1254*m*, 1170*m*. |
| **$^1$H-NMR** (300 MHz, $d_6$-DMSO): | 0.85-1.38 (*m*, 2H, H-C(4) H-C(6)); 1.27 (*s*, 9H, $C(CH_3)_3$); 1.62-2.00 (*m*, 5H, 2H-C (3), H'-C(4), H-C(5), H'-C(6)); 1.73 (*s*, $CH_3$-C(5')); 2.15 ($m_c$, 2H, C$H_2$COOH); 3.75 ($m_c$, H-C(1)); 4.19 ($m_c$, H-C(2)); 6.77 (*d*, *J*(H-N, H-C(1)) = 9.6, NH); 7.56 (s, H-C (6')); 11.09 (*s*, H-N(3')); 12.08 (br. *s*, $CH_2$COO*H*). |

Im Bereich von 6.00-10.0 ppm sind mehrere kleine Signale zu erkennen, die beim Auf-heizen der NMR-Probe auf 80°C verschwinden.

Die Zuordnung der Signale erfolgte mit Hilfe eines $^1$H, $^1$H-*COSY*-Spektrums.

| | |
|---|---|
| **$^{13}$C-NMR** (75.5 MHz, $d_6$-DMSO): | 12.03 (*C*$H_3$-C(5')); 27.90 (C(*C*$H_3$)$_3$); 29.16 (C(3)); 30.72 (C(4); 32.72 (C(5)); 38.06 (C(6)); 40.30 (*C*$H_2$COOH); 49.71 (C(1)); 57.05 (C(2)); 77-51 (*C*($CH_3$)$_3$); 107.65 (C(5')); 138.32 (C(6')); 151.28, 154.79 ($O_2$*C*N, C(2')); 163.60 (C(4')); 173.35 ($CH_2$*C*OOH). |

Die Zuordnung der Signale erfolgte mit Hilfe eines $^1$H, $^{13}$C-*COSY*-Spektrums.

| | | |
|---|---|---|
| **MS** (ESI'): | 382.3 [M+H]. | |
| **Anal.**: | ber. für $C_{18}H_{27}N_3O_6$ (381.43): | C 56.68, H 7.13, N 11.02; |
| | gef.: | C 56.45, H 7.16, N 10.80. |

Beispiel 2

*Synthese eines kopplungsjähigen Adeninbausteins*

**Herstellung von 9-[(*S*,*S*,*S*)-8-Azabicyclo[3.3.1]nonan-7-on-2-yl]-6-*N*-benzoyl-adenin (15a)**

**1. Durchführung**

Ein 500 ml Zweihalsrundkolben wurde mit 180 ml trockenem DMF und 2.25 g einer Suspension an NaH (60 mmol) beladen. 7.18 g 6-*N*-Benzoyladenin (30 mmol) wurden portionsweise hinzugegeben und and die Mischung solange geführt bis die Gasentwicklung abklang. Nach 15 min wurden 7.95 g Iodlactam 9 (30 mmol) hinzugegeben und die Lösung im Dunklen 22 h lang gerührt. Danach wurde die Lösung auf 0°C abgekühlt und durch Zugabe von 1 M wäßriger HCl (24 ml) neutralisiert. Unter reduziertem Druck (0.3 mbar, Badtemperatur 60°C) wurde das Lösemittel entfernt. Der resultierende Rest wurde in 100 ml MeOH gelöst und auf 15 g Silicagel vorabsorbiert. Eine Chromatographie über 7 x 15 cm Silicagel mit $CH_2Cl_2$/MeOH (12:1) ergab 17 g eines gelben Öls, welches in 15 ml MeOH umkristallisiert wurde, um 8.2 g **15a** (22 mmol; 73%) eines farblosen Feststoffes zu ergeben, der für weitere Reaktionen rein genug war. Eine analytische Probe wurde durch Kristallisation aus MeOH/$Et_2O$ erhalten.

**2. Analytische Daten**

| | |
|---|---|
| Schmp.: | 269-271°C (Zersetzung) |
| TLC: | $CH_2Cl_2$/MeOH 10: 1, $R_f$ 0.28 |
| $[a]_D^{20}$: | +35.9° (*c* 1.03, MeOH) |
| UV (MeOH): | $l_{max}$ 280 (20329) |
| IR: | 3500-3000*s*, 2938*m*, 1685*m*, 1654*s*, 1610*s*, 1542*w*, 1508*m*, 1490*m*, 1458s, 1400*m*, 1341*m*, 1286*s*, 1254*s*, 1168*w*, 1098*m*, 799*w*, 713*m*, 643*w*. |
| $^1$H-NMR (300 MHz, $d_6$-DMSO): | 1.53-1.72 (*m*, 3H); 2.08-2.32 (*m,* 5H); 2.48-2.58 (*m*, 1H); 4.27 (br. *m*, 1H, H-C(1')); 4.63 (br. *m,* 1H, H-C(2')); 7.52-7.58 (*m*, 2H, Ph); 7.62-7.68 (*m*, 1H, Ph); 8.04-8.08 (*m*, 2H, Ph); 8.14 (d, *J* (H-N(8'), H(C(1')) = 4.3 Hz, H-N(8')); 8.70, 8.75 (2 s, 2H, H-C(2); H-C-(8)); 11.18 (s, 1H, H-NC(6)). |
| $^{13}$C-NMR (75 MHz, $d_6$-DMSO): | 18.89, 24.21, 27.27 (C(3'), C(4'), C(9')); 25.15 (C(5')), 36.95 (C(6')); 47.44 (C(1')); 54.21 (C(2')); 125.32 (C(5)), 128.34, 132.28, 133.34 (Ph); 143.05 (C(8)); 150.17 (C(4)); 151.12 (C(2)); 152.59 (C(6)); 165.49 (Bz-C=O); 171.39 (Lactam-C=O). |
| MS ($ESI^-$): | 377 [M+H] (100%) |
| Mikroanalyse: | kalkuliert für $C_{20}H_{20}N_6O_2$: C 63.83, H 5.32, N 22.34<br>gefunden: C 63.90, H 5.45, N 22.1 |

**Herstellung von 9-[(*S*,*S*,*S*)-8-Azabicyclo[3.3.1]nonan-7-on-2-yl]-adenin (15b)**

**1. Durchführung**

Ein 500 ml Rundhalskolben wurde mit 8.2 g Benzoyl-A-lactam **15a** (22 mmol) und 250 ml trockenem Methanol beladen. Die Suspension wurde erhitzt bis **15a** vollständig aufgelöst war. Danach wurde eine Lösung von 1.62 g NaOMe in 5.5 ml MeOH hinzugegeben und die Mischung 19 h lang bei Raumtemperatur gerührt. Die resultierende Suspension wurde filtriert und das Präzipitat mit $Et_2O$ gewaschen. Das Filtrat wurde durch Zugabe von Ionenaustauscherharz (Amberlite IR-120, H'-Form) neutralisiert. Das Harz wurde abfiltriert und das Lösemittel unter reduziertem Druck entfernt. Der Rückstand wurde in 4 ml MeOH gelöst und durch Zugabe von 30 ml $Et_2O$ ausgefällt. Das Präzipitat wurde gefiltert und die vereinten Präzipitate unter Vakuum getrocknet, um 5.8 g **15b** (21 mmol, 97%) zu ergeben, das rein genug für weitere Reaktionen war. Eine analytische Probe wurde durch Kristallisation aus MeOH/$H_2O$ erhalten.

**2. Analytische Daten**

| | |
|---|---|
| Schmp.: | 300-302°C (Zersetzung) |
| TLC: | MeOH, $R_f$ 0.35 |
| $[a]_D^{20}$: | +42.8° (c 0.40, MeOH) |
| UV (MeOH): | $l_{max}$ 260 (16374) |
| IR: | 3367*s*, 3179*s*, 3101*m*, 2926*m*, 2864*w*, 1651*s*, 1600*s*, 1563*m*, 1470*m*, 1337*m*, 1296*m*, 1258*m*, 1229*m*, 1166*w*, 1107*w*, 1005*w*, 802*w*, 724*w*, 668*w*. |
| $^1$H-NMR (300 MHz, $d_6$-DMSO): | 1.48-1.67 (*m*, 3H); 2.04-2.24 (*m*, 5H); 2.46-2.54 (*m*, 1H); 4.20 (br. *m*, 1H, H-C(1')); 4.46 (br. *m*, 1H, H-C(2')); 7.26 (*s*, 2H, $NH_2$); 8.06 (*d*, *J* (H-N(8'), H(C(1')) = 4.3 Hz, H-N(8')); 8.14, 8.35 (2*s*, 2H, H-C(2); H-C-(8)). |
| $^{13}$C-NMR (75 MHz, $d_6$-DMSO): | 18.94, 24.15, 27.36 (C(3'), C(4'), C(9')); 25.20 (C(5')); 37.03 (C(6')); 47.47 (C(1')); 53.85 (C(2')); 118.75 (C(5)); 139.18 (C(8)); 149.70 (C(4)); 152.15 (C(2)), 156.03 (C(6)); 178.91 (lactame-C=O). |
| MS (ESI$^+$): | 273 [M+H] (100%) |
| Mikroanalyse: | kalkuliert für $C_{13}H_{16}N_6O$ x $H_2O$: C 53.78, H 6.25, N 28.95<br>gefunden: C 53.65, H 6.24, N 29.12 |

**Herstellung von 9-[(*S,S,S*)-8-Azabicyclo[3.3.1]nonan-7-on-2-yl]-6-*N*-dimethylaminomethylidene-adenin (15c)**

**1. Durchführung**

Ein 500 ml Rundkolben wurde mit 5.58 g A-lactam **15b** (20 mmol), 200 ml trockenem DMF und 17.1 ml Dimethylformamiddiethylacetal (100 mmol) beladen. Die Mischung wurde auf 80°C 3h lang erhitzt. Die resultierende klare Lösung wurde unter reduziertem Druck (0.4 mbar, 60°C Badtemperatur) konzentriert, um 6.9 g von rohem **15c** zu ergeben, das ohne weitere Reinigung verwendet wurde.

**2. Analytische Daten**

| | |
|---|---|
| TLC ($Al_2O_3$): | $CH_2Cl_2$/MeOH 20:1, $R_f$ 0.75 |
| $^1$H-NMR (300 MHz, $CDCl_3$): | 1.58-1.79 (*m*, 2H); 1.89-1.93 (*m*, 1H); 2.16-2.28 (*m*, 2H); 2.38-2.51 (*m*, 3H); 2.69-2.78 (*m*, 1H); 3.23, 3.28 (2 s, 6H, $CH_3$); 4.38 (br. *m*, 1H, H-C(1')); 4.68 (br. *m*, 1H, H-C(2')); 6.62 (d, *J* (H-N(8'), H(C(1')) = 4.3 Hz, H-N(8')); 8.13, 8.54 (2 *s*, 2H, H-C(2); H-C-(8)); 8.97 (*s*, 1H, Formamidin-H). |
| MS (ESI$^-$): | 328 [M+H] (100%) |

**Herstellung von 9-[(*S,S,S*)-8-*N*-*Tert*.-butoxycarbonyl-8-azabicyclo[3.3.1]nonan-7-on-2-yl]-6-*N*-dimethylaminomethylidene-adenin (15d)**

**1. Durchführung**

Ein 250 ml Rundkolben wurde mit 6.9 g rohem Amidine-A-lactame **15c** (20 mmol) und 100 ml trockenem $CH_2Cl_2$ beladen. Unter Rührem wurden 2.8 ml Triethylamin (20 mmol) hinzugegeben, gefolgt von 8.7 g $Boc_2O$ (40 mmol) und 2.44 g DMAP (20 mmol). Die Lösung wurde 16 h lang bei Raumtemperatur gerührt bis das gesamte Ausgangsmaterial verbraucht war. Das Lösemittel wurde unter reduziertem Druck entfernt und der resultiernde Rückstand wurde mittels Chromatographie (7 x 16 cm Silicagel, Elutionsmittel $CH_2Cl_2$/eOH 20:1) gereinigt, um 8.2 g eines gelben Feststoffes zu ergeben, der in einen 100 ml Rundkolben überführt wurde. Danach wurden 50 ml $Et_2O$ hinzugegeben und die Mischung wurde 2 h lang unter Rückfluß beschallt. Nach dem Abkühlen auf Raumtemperatur wurde die Mischung filtriert und das Präzipitat wurde mit $Et_2O$ behandelt und noch einmal mit Ultraschall beschallt. Die Filtration ergab 6.66 g **15d** (78%) eines leicht gelblichen Feststoffes, der rein genug für weitere Reaktionen war.

**2. Analytische Daten**

| | |
|---|---|
| TLC: | $CH_2Cl_2$/MeOH 10:1, $R_f$ 0.66 |
| $^1$H-NMR (300 MHz, $CDCl_3$): | 1.54 (*s*, 9H, *tert.*-Bu); 1.84-2.36 (*m,* 7H); 2.44 (*d*, 1H, *J* = 18.5 Hz); 2.77 (*dd*, *J* = 7.0, 18.5 Hz, 1H); 3.14, 3.20 (2 *s*, 6H, Formamidin-$CH_3$); 4.80 (br. *m*, 1H, H-C(1')); 5.23 (br. *m*, 1H, H-C(2')); 8.04, 8.48 (2 s, 2H, H-C(2); H-C-(8)); 8.87 (*s*, 1H, Formamidin-H). |
| MS (ESI$^+$): | 428 [M+H] (100%) |

**Herstellung von 9-[(*S*,*S*,*S*)-8-*N*-*Tert*-butoxycarbonyl-8-azabicyclo[3.3.1]nonan-7-on-2-yl]-adenin (15e)**

**1.Durchführung**

Ein 250 ml Rundkolben wurde mit 6.64 g Amidine-A-lactame **15d** (15.5 mmol) und 150 ml trockenem $CH_2Cl_2$ beladen. Danach wurden 11.55 g of *p*-Toluolsulfonsäurehydrazid (62.0 mmol) hinzugegeben gefolgt von 1.47 g TsOH (7.75 mmol). Die Lösung wurde bei Raumtemperature 44 h lang gerührt. Nach der Zugabe von 150 ml $CH_2Cl_2$ wurde die Lösung dreimal mit Wasser gewaschen und durch Zugabe von 2 M NaOH auf einen pH > 12 eingestellt. Die vereinigten Waschlösungen wurden mit 100 ml $CH_2Cl_2$ extrahiert und die vereinigten organischen Phasen über $MgSO_4$ getrocknet. Nach der Filtration wurde das Lösemittel unter reduziertem Druck entfernt und der resultierende Rückstand mittels Chromatographie (5.2 x 20 cm Silicagel, Elutionsmittel: $CHCl_3$/MeOH 30:1) gereinigt, um 4.96 g **15e** (86%) zu ergeben, das für weitere Reaktionen rein genug ist. Eine analytische Probe wurde durch Kristallisation aus THF/Cyclohexan erhalten.

**2. Analytische Daten**

| | |
|---|---|
| Schmp.: | 211 °C (gas evol.) 300-302°C (Zersetzung) |
| TLC: | CHC[$_3$/MeOH 30:1, $R_f$ 0.23 |
| [a]$_D^{20}$: | +80.6° (*c* 1.32, $CH_2Cl_2$) |
| UV (MeCN/$H_2O$ 1:9): | $l_{max}$ 260 (14136) |
| IR: | 3406*m*, 3328*m*, 3206*m*, 2941*w*, 1756*s*, 1724*s*, 1701*m*, 1668*s*, 1645*s*, 1597*s*, 1570*m*, 1479*m*, 1414*w*, 1304*m*, 1273*s*, 1251*s*, 1228*m*, 1145*s*, 1034*w*, 996*w*, 853*w*, 760*w*. |
| $^1$H-NMR (300 MHz. $CDCl_3$): | 1.62 (*s*, 9H, *tert.*-Bu); 1.81-1.89 (*m*, 3H); 2.19-2.37 (*m*, 4H); 2.54 (*d*, *J* = 18.5 Hz. 1H); 2.86 (*dd*, *J* = 7.0, 18.5 Hz, 1H); 4.86 (br. *m*, 1H, H-C(1')); 5.28 (br. *m*, 1H, H-C(2')); 5.64 (*s*, 2H, $NH_2$); 8.06, 8.38 (2 *s*. 2H. H-C(2); H-C-(8)). |
| $^{13}$C-NMR (75 MHz, $d_6$-DMSO): | 19.93, 25.45, 27.23 (C(3'), C(4'), C(9')); 25.97 (C(5')); 28.01 (C($\underline{C}H_3)_3$)); 40.24 (C (6')); 52.65 (C(1')); 53.68 (C(2')); 84.12 ($\underline{C}(CH_3)_3$)); 119.82 (C(5)); 139.20 (C(8)); 150.59 (C(4)); 152.58 (C(2)); 155.55 (C(6)); 158.46 (Boc-C=O); 171.14 (lactame-C=O). |
| MS (ESI$^-$): | 373 [M+H] (100%) |
| Mikroanalyse: | kalkuliert für $C_{18}H_{24}N_6O_3$: C 58.05, H 6.50, N 22.57 |
| | gefunden: C 58.13, H 6.51, N 22.85 |

**Herstellung von 9-[(*S*,*S*,*S*)-8-*N*-*Tert.*-butoxycarbonyl-8-azabicyclo[3.3.1]nonan-7-on-2-yl]-6-*N*-p-methoxybenzoyl-adenin (15f)**

**1.Durchführung**

Ein 100 ml Zweihalsrundkolben wurde mit 4.96 g Boc-A-lactame **15e** (13.3 mmol) und 60 ml trockenem $CH_2Cl_2$ beladen. Danach wurden 5.35 ml trockenes Pyridin (66.5 mmol) hinzugegeben, gefolgt von 1.59 g DMAP (1.3 mmol). Nach dem Abkühlen auf 0°C wurden 5.4 ml 4-Methoxybenzoylchlorid (39.9 mmol) tropfenweise hinzugegeben und die Mischung wurde bei 0°C 15 min lang gerührt. Das Eisbad wurde entfernt und die Reaktionsmischung wurde 22 h lang bei Raumtemperatur gerührt. Danach wurde die Reaktionsmischung wieder auf 0°C gekühlt und 35 ml MeOH wurden tropfenweise hinzugegeben. Nach 30 min bei 0°C wurden 80 ml einer gesättigten Lösung von $NH_3$ in MeOH tröpfchenweise hinzugegeben. Es bildete sich ein weißes Präzipitat, das sich nach vollständiger Zugabe der Lösung auflöste. Nach 30 min wurde das Eisbad entfernt und die Reaktion bei Raumtemperatur für weitere 2 h gerührt. Anschließend

wurde das Lösemittel unter reduziertem Druck entfernt und der resultierende Rückstand in 200 ml $CH_2Cl_2$ aufgelöst. Die Lösung wurde nacheinander mit 150 ml gesättigter $NaHCO_3$-Lösung und wäßriger Zitronensäurelösung (20%, 2 x 100 ml) gewaschen, über $MgSO_4$ gewaschen, filtriert und im Vakuum konzentriert. Der resultierende Rückstand wurde mittels Chromatographie (5.2 x 18 cm Silicagel; Elutionsmittel: EtOAc/MeOH 40:3) gereinigt, um 5.93 g **15f** (88%) zu ergeben.

**2. Analytische Daten**

| | |
|---|---|
| Schmp.: | 110-112°C (Zersetzung) |
| TLC: | $CH_2Cl_2$/MeOH 40:1, $R_f$ 0.44 |
| $[a]_D^{20}$: | +58.3° (*c* 1.32, $CH_2Cl_2$) |
| UV (MeOH): | $l_{max}$ 288 (30459) |
| IR: | 3600-3050*m*, 2940*m*, 1757*s*, 1707*m*, 1671*m*, 1604*s*, 1577*m*, 1506*s*, 1458*m*, 1402*m*, 1342*m*, 1251*s*, 1168*m*, 1145*s*, 1100*m*, 1024*m*, 893*w*, 848*m*, 794*w*, 761*m*, 644*w*. |
| $^1$H-NMR (300 MHz, $CDCl_3$): | 1.63 (*s*, 9H, *tert.*-Bu); 1.84-1.91 (*m*, 3H); 2.24-2.40 (*m*, 4H); 2.55 (*d*, *J* = 18.5 Hz, 1H); 2.88 (*dd*, *J* = 7.0, 18.5 Hz, 1H); 3.92 (*s*, 3H, OMe); 4.95 (br. *m,* 1H, H-C(1')); 5.28 (br. *m,* 1H, H-C(2')); 7.01-7.04 (*m,* 2H, PMBz-H); 8.01-8.04 (*m,* 2H, PMBz-H); 8.26, 8.81 (2 *s*, 2H, H-C(2); H-C-(8)); 8.91 (s, 1H, H-NC(6)). |
| $^{13}$C-NMR (75 MHz, $CDCl_3$): | 19.95, 25.55, 27.24 (C(3'), C(4'), C(9')); 25.95 (C(5')); 28.06 (C($\underline{C}H_3$)$_3$)); 40.28 (C(6')); 52.61 (C(1')); 53.99 (C(2')); 55.54 (OMe); 84.27 ($\underline{C}(CH_3)_3$)); 114.08 (PMBz); 123.12 (C(5)); 126.24 (PMBz); 130.03 (PMBz); 141.58 (C(8)); 149.87 (C(4)); 152.00 (C(2)); 152.31 (C(6)); 152.54, 163.31, 164.02 (PMBz, PMBz-C=O, Boc-C=O); 171.14 (Lactam-C=O). - |
| MS (ESr): | 507 [M+H] (100%) |
| Mikroanalyse: | kalkuliert für $C_{26}H_{30}N_6O_5$: C 61.66, H 5.93, N 16.60 gefunden: C 61.75, H 6.01, N 16.69 |

**Herstellung von9-((*S*,*S*,*S*)-2-(*Tert.*-butoxycarbonyl)amino-4-(carboxymethyl)cyclohexyl]-6-*N*-*p*-methoxybenzoyl-adenin (16)**

**1. Durchführung**

Ein 500 ml Zweihalsrundkolben wurde mit 5.25 g PMBzBoc-A-lactam **15f** (10.36 mmol) und 200 ml THF beladen. Nach dem Abkühlen auf 0°C, wurde eine Lösung von 2.17 g LiOH x $H_2O$ (51.8 mmol) in 50 ml $H_2O$ über einen Zeitraum von 20 min. tropfenweise hinzugegeben. Danach wurden 30 ml MeOH hinzugegeben, das Eisbad wurde entfernt und die Reaktionsmischung wurde für eine 1h bei Raumtemperatur gerührt. Danach wurde ein Ionenaustauscherharz (Amberlite IR-120, $H^+$-Form) hinzugegeben bis ein pH von 7 erreicht wurde. Das Harz wurde durch Filtration entfernt und die Lösung wurde unter reduziertem Druck auf ein Volumen von 100 ml konzentriert. Anschließend wurden 200 ml $H_2O$ hinzugegeben und ein pH von 2 durch Zugabe von 1 M wäßriger HCl eingestellt. Die Lösung wurde mit EtOAc (3 x 200 ml) extrahiert und die vereinigten organischen Extrakte über $MgSO_4$ getrocknet, filtriert und im Vakuum konzentriert. Der resultierende Rückstand wurde in 25 ml von heißem MeOH gelöst. Das Produkt wurde anschließend durch Zugabe von 10 ml $H_2O$ ausgefällt. Das Präzipitat wurde filtriert, mit $H_2O$ gewachen und über $P_4O_{10}$ getrocknet, um 1.95 g 16 zu ergeben. Weitere 0.57 g konnten von der Mutterlösung und den Waschlösungen erhalten werden, um eine Gesamtausbeute von 46% zu ergeben.

**2. Analytische Daten**

| | |
|---|---|
| Schmp.: | 238-239°C |
| TLC: | $CH_2Cl_2$/MeOH 10:1, $R_f$0.39 |
| $[a]_D^{20}$: | +19.9° (*c* 0.55, MeOH) |
| UV (MeCN/$H_2O$): | $l_{max}$ 290 (22722) |
| IR: | 3368*m*, 2939*w*, 1710*m*, 1683*s*, 1608*s*, 1577*m*, 1528*m*, 1507*m*, 1458*m*, 1306*m*, 1250*s*, 1175*s*, 1030*w*, 842*w*, 761*w*. |

(fortgesetzt)

| | |
|---|---|
| $^{1}$H-NMR (300 MHz,$d_6$-DMSO): | 1.08 (*s*, 9H, *tert.-Bu*); 1.09-1.40 (*m*, 2H); 1.80-2.35 (*m*, 7H); 3.86 (*s*, 3H, OMe); 4.07, 4.36 (2 br. *m*, 2H, H-C(1') H-C(2')); 6.87(br. s, 1H, NHBoc); 7.06-7.09 (m, 2H, PMBz-H); 8.00-8.05 (m, 2H, PMBz-H); 8.42, 8.86 (2 s, 2H, H-C(2); H-C-(8)); 10.88 (s, 1H, H-NC(6)). |
| $^{13}$C-NMR (75 MHz, $CDCl_3$): | 27.73 (C($\underline{C}H_3)_3$)); 30.35, 30.46, 37.95 (C(3'), C(5'), C(6')); 32.89 (C(4')); 40.32 ($\underline{C}H_2CO_2H$); 51.30 (C(2')); 55.38 (OMe); 77.45 ($\underline{C}(CH_3)_3$)); 113.55 (PMBz); 125.24 (C(5)); 125.63 (PMBz); 131.41 (PMBz); 143.48 (C(8)); 150.03 (C(4)); 150.69 (C(2)); 152.38 (C(6)); 154.53, 162.38, 164.77 (PMBz, PMBz-C=O, Boc-C=O); 173.36 ($CO_2H$) |
| MS ($ESI^-$): | 525 [M+H] (100%) |
| Mikroanalyse: | kalkuliert für $C_{26}H_{32}N_6O_6$: C 59.53, H 6.15, N 16.02<br>gefunden: C 59.3 5, H 6.32, N 15.89 |

Beispiel 3

*Synthese eines Oligomers mit der Sequenz A TA TA*

Als polymerer Träger wird ein Polyoxyethylen-(POE)-Polystyrol-Copolymer (Tentagel S HMB, 0,23 mmol/g) verwendet, das sowohl in wäßriger Lösung als auch in organischen Lösungsmitteln gute Quelleigenschaften besitzt. Die Aminoethylfunktionen des Polymers sind mit einem Hydroxymethyl-benzoyl-(HMB)-Linker derivatisiert; die Beladung mit dem ersten A-Baustein erfolgt unter Verwendung eines 5-fachen Überschusses nach der Methode des symmetrischen Anhydrids (Zugabe von 2.5 eq. DIC und 2.5 eq. DMAP) innerhalb von 20 h in DCM.

Es erfolgte schrittweise Verlängerung der Oligomerkette durch repetitive Zyklen, die aus Abspaltungen der temporären Boc-Schutzgruppen und Kupplungen der Boc-geschützten Monomer-Bausteine bestehen. Am Ende der Synthese wird die N-terminale Boc-Schutzgruppe mit 50% TFA/DCM abgespalten, das Oligomer wird mit 2 N HCl/MeOH vom HMB-Linker gespalten und mit HPLC für die weiteren Umwandlungsexperimente aufgereinigt.

Der Standard-Kupplungszyklus ist in der Tabelle beschrieben: die Boc-Schutzgruppe der Aminofunktion wird mit 50% TFA/DCM abgespalten (30. min), anschließend wird das Harz mit DCM gewaschen und mit 1 M DIEA/DMF neutralisiert und mit DMF aminfrei gewaschen. Die Kupplungen erfolgen jeweils nach Voraktivierung des Monomer-Bausteins (3 eq.) mit HATU (3 eq.) in DMF und unter Zusatz von 1 M DIEA/DMF (6 eq.) und 2 M Lutidin/DMF (12 eq.). Die Kupplungszeiten betragen 3 h bei Raumtemperatur. Nach Beendigung der Kupplungszyklen werden nicht-umgesetzte Aminofunktionen mit Essigsäureanhydrid (20 eq.) unter Pyridinzusatz (10 eq.) gecappt. Zur Reaktionskontrolle mittels HPLC wurden nach jedem Kupplungsschritt ein paar Harzkügelchen entnommen, die zur Abspaltung der Boc-Schutzgruppe mit 50% TFA/DCM behandelt wurden. Dann wurde das jeweilige Oligomer mit 2 N NaOH/MeOH (15 min, RT) abgespalten, die Abspaltlösung wurde mit HCl neutralisiert und per RP-HPLC analysiert.

Nach Beendigung der Synthese erfolgt die Abspaltung der Boc-entschützten Oligomeren vom HMB-Harz, wie gewohnt, mit 2 N NaOH/MeOH (15 min, RT). Die Abspaltung der p-Methoxybenzoyl-Schutzgruppen bei ($A^{PMBz}$)-haltigen Oligomeren wird dadurch erreicht, daß die Abspaltungslösung 2.5 h bei 55 °C gehalten wird. Die Aufreinigung erfolgt nach der Neutralisation der Abspaltungslösung mit HCl durch RP-HPLC. Die isolierten HPLC-Fraktionen der Syntheseprodukte wurden mit analytischer HPLC auf ihre Reinheit überprüft, mit Elektrospray(ESI)-Massenspektrometrie eindeutig charakterisiert, und eine reversible UV-Umwandlungskurve bei 265 nm ($C_{ATATA}$=27,9 μM in 5 mM Tris/HCl; pH 7,0) aufgenommen (Fig. 4.), womit die Eignung als Paarungssystem bewiesen ist.

**ATATA:**

Mber= 1361,0 (M+H)gef= 1362,0
tR = 14,1 min (10 bis 40 % Acetonitril in 0,1 %iger TFA in Wasser in 30 min)
Absorptionsmaximum: 263 nm

Tabellarische Übersicht zu den Kupplungen:

| Operation | Reagenz/Lsg.mittel | Volumen | Zeit |
|---|---|---|---|
| 1. Deblockierung | 50% TFA/DCM | 150 µl | 1 x 5 min<br>1 x 25 min |
| 2. Waschen | DCM | 150 µl | 5 x (im Durchfluß) |
| 3. Neutralisation | 1 M DIEA/DMF | 75 µl | 1 x (im Durchfluß) |
| 4. Waschen | DMF | 150 µl | 5 x (im Durchfluß) |
| 5. Kupplung | Baustein/HATU/DMF | 80 µl | 180 min (Schütteln) |
| | 1 M DIEA/DMF | 12 µl | |
| | 2 M Lutidin/DMF | 12 µl | |
| 6. Waschen | DMF | 150 µl | 3 x (im Durchfluß) |
| 7. Analytik mit HPLC: Probeabspaltung mit 2 N NaOH/MeOH (15 min,RT) | | | |
| 8. Capping | $Ac_2O$ | 10 µl | 20 min (Schütteln) |
| | Pyridin | 10 µl | |
| | DCM | 100 µl | |
| 9. Waschen | DCM | 150 µl | 5 x (im Durchfluß) |

Beispiel 4

*Reversible Bildung eines homodimeren Peptids*

Analog .Beispiel 3 wird eine Sequenz AATAT an der Festphase aufgebaut, und an diesen Paarungsabschnitt ein Boc-Lysin(Fmoc)-Baustein gekuppelt. Mit 40 % Piperidin in DMF wird die Fmoc-Gruppe abgespalten, mit DMF gewaschen und dann mit Jodessigsäure (20 eq.) und DIC (20 eq.) in DMF versetzt. Nach 15 h bei RT. wird die Lösung abfiltriert, das Harz mit DMF gewaschen und mit dem Peptid CYSKVG (50 eq.) in DMF versetzt. Man beläßt 15 h bei RT., filtriert die Lösung ab und wäscht das Harz mit DMF und MeOH. Man spaltet mit 2 M NaOH wäßrig mit MeOH (1: 1) vom Harz ab und erhitzt die Abspaltlösung 2.5 h auf 55 °C. Nach Abkühlen neutralisiert man mit 2 M Salzsäure und isoliert das Produkt mit RP-HPLC (10 bis 40 % Acetonitril in 0,1 %iger TFA in Wasser in 30 min). Die Produktfraktion (V = 3 ml) wird im Vakuum vom Acetonitrilanteil befreit und auf 500 µl wäßrige Lösung eingeengt. Diese Lösung wurde über eine SepPak Plus C18-Cartridge (Waters) entsalzt, und auf 1 ml wäßrige Lösung eingestellt. Die Konzentration des Oligomers betrug c = 140 µmol/l (aus E = 1,004 bei 265 nm, gemessen bei 70 °C).

Die UV-Umwandlungskurve einer 10 µmolaren Lösung zeigte, daß unterhalb der Raumtemperatur die Substanz als einheitliches Homodimer vorliegt, während oberhalb von 40 °C kein Homodimer nachgewiesen werden kann. Dieses Gleichgewicht ist reversibel, der Anteil an Homodimer kann durch Wahl der Temperatur und der Konzentration eingestellt werden.

Beispiel 5

Übersicht der thermodynamischen Daten selbstkomplementärer CNA-Oligomere

| Sequenz | Basenpaare | Stacks Purin-Purin | Purin-Pyrimidin | $T_m$ (1 µm) [°C] | $T_m$ (10µm) [°C] | ΔG (298K) [kcal/mol] | ΔH [kcal/mol] | ΔS [cal/molK] |
|---|---|---|---|---|---|---|---|---|
| A T A T A | 4 | 3 | 0 | 20.9 | 32.3 | -7.52 | -36 | -95 |
| A A T A T | 4 | 2 | 2 | 27.4 | 40.5 | -8.47 | -33 | -82 |
| A A A T T | 4 | 1 | 4 | 35.0 | | -9.78 | -49 | -130 |
| T T A A A | 4 | 0 | 2 | 10.0 | | -6.6 ± 0.5 | -33 | -90 |
| A T A T A T | 6 | 3 | 0 | 22.5 | | | | |
| A T A T | 4 | 2 | 0 | | = -2° | -4.3 | -24 | -65 |

Beispiel 6

Konjugation mit einem definierten Peptid oder einer Peptidbibliothek

Die Konjugation von definierten Peptiden und Peptidbibliotheken an CNA wurde unter identischen experimentellen Bedingungen durchgeführt. Im folgenden wird die Konjugation der CNA Ac-CNA(AATAT)-Lys-OH **(1)** mit dem Peptid H-Cys-Ser-Lys-Val-Gly-OH (3) beschrieben.

6.1 Iodacetylierung von (1) an der ε-Aminofunktion von Lysin

**1** (0,69 μmol) wurde in 800 μl 0.1 M Bicarbonat-Lösung gelöst und mit 150 Equivalenten Iodessigsäure-N-succinimidylester (102 μmol, 28,9 mg) in 400 μl Dimethylsulfoxid 2 h unter Lichtausschluß geschüttelt. Das Produkt Ac-CNA(AATAT)-Lys($N^{\varepsilon}$-Iodacety)-OH **(2)** wurde direkt über präparative RP-HPLC aufgereinigt und über eine RP-C18 Kartusche entsalzt analytische RP-HPLC: $R_t$ = 17,93 min
ESI-MS: M,(ber) = 1699,7, $M_r$(gef) = 1699,4

6.2 Konjugation von **2** mit H-Cys-Ser-Lys-Val-Gly-OH 3

Zur Lösung von **2** (106 nmol) in 20 μl Wasser/Dimethylformamid (1:1) wurde eine Lösung von **3** (127 nmol, 83 μg, Gehalt ca. 70 %) in 30 μl 0,5 M Phosphatpuffer, 20 mM EDTA pH 6.0 gegeben und der Ansatz wurde unter Lichtausschluß geschüttelt. Nach 1 h hatte das Peptid zwar vollständig abreagiert, die CNA war jedoch nur zu ca. 50% umgesetzt. Durch nochmalige Zugabe derselben Menge an Peptid in Puffer und 1 h Schütteln unter Lichtausschluß wurde 2 vollständig umgesetzt. Das CNA-Peptid Konjugat wurde direkt über RP-HPLC aufgereinigt und über eine RP-C 18 Kartusche entsalzt.
analytische RP-HPLC: $R_t$ = 16,93 min
ESI-MS: M,(ber) = 2227,6, $M_r$(gef) = 2227,4

Beispiel 7

Verbesserung der Löslichkeit durch Phosphatierung

7.1 Allgemeine Vorschrift zur Phosphatierung von CNA:

7.1.1 Kupplung von DMT-geschützter 4-Hydroxybuttersäure (Hba) an AATAT-HMB-Harz

100 mg AATAT-HMB-Harz mit einer Belegung von 0,15 mmol/g Harz (0.015 mmol) wurden nach der Abspaltung der letzten Boc-Schutzgruppe in einer 5 ml Kunststoffspritze mit Fritte mit 5 ml 1 M DIPEA-Lösung in DMF neutralisiert und 4 mal mit DMF gewaschen. 0,15 mmol (61 mg) DMT-Hba (M = 406,48) und 0,15 mmol (57 mg) HATU (M = 380,2) wurden in 2 ml NMP gelöst und geschüttelt. Nach 10 Minuten wurden 0,9 mmol DIPEA (900 μl einer 1 M Lösung in DMF) zugegeben und geschüttelt. Die Kupplungslösung wurde zum Harz gegeben und die Spritze mit dem Harz 4 h um die kurze Spritzenachse gedreht (ca. 0,5 Umdrehungen/s), um für eine gute Durchmischung zu sorgen. Die Lösung wurde aus der Spritze gedrückt und das Harz 4 mal mit 4 ml DMF und 2 mal mit 4 ml DCM gewaschen.

7.1.2 Detritylierung

Das Harz wurde 5 mal mit 4 ml 6 % DCA in DCM je 2 Minuten detrityliert und 4 mal mit DCM und 3 mal mit trockenem Acetonitril (ACN) nachgewaschen. Das Harz wurde über Nacht im Exsikkator über $P_2O_5$ getrocknet.

7.1.3 Phosphitylierung und Oxidation

Das Harz wurde unter Argon mit 2 ml 0,5 M Pyridiniumhydrochlorid in trockenem ACN gequollen. Es wurden 10 eq. (0.15 mmol, 41 mg) Bis-(2-cyanoethyl)-N,N-Diisopropylamino-Phosphoramidit (M = 271,3) zugegeben und 20 Minuten gedreht. Das Harz wurde 4 mal mit 4 mL trockenem ACN gewaschen und mit 0,5 ml einer 6 M t-Bu00H Lösung in Decan und 1 mL ACN 30 Minuten oxidiert. Anschließend wurde 4 mal mit trockenem ACN nachgewaschen.

7.1.4 Abspaltung der Cyanoethylschutzgruppen

Das Harz wurde mit 4 ml einer 2 M Lösung von DBU in Pyridin versetzt und 15 h gedreht. Danach wurde 6 mal

mit ACN gewaschen.

7.1.5 Abspaltung vom Harz und pMBz-Entschützung

Mit 2 ml 2M NaOH in Wasser wurde die Substanz innerhalb von 10 Minuten vom Harz gespalten. Es wurde mit weiteren 2 ml Natronlauge nachgewaschen und die resultierende Lösung 3 h auf 55 °C gehalten. Nach Neutralisation mit 2 M HCl wurde die Verbindung an der RP-HPLC gereinigt (Eluent Wasser/Acetonitril mit 0,1 % TFA).

7.1.6

Zuletzt wurde die Substanz wie bereits beschrieben an einer SEP PAK Kartusche (RP) entsalzt und bei RT und reduziertem Druck bis zur Trockene eingeengt.
Massenspektrum von $H_2PO_3$-Hba-AATAT ($M_{exakt}$ = 1526,7).
Sichtbar ist das Molekülion mit Na-Clustern einfach und zweifach geladen.
Die Umwandlungskurve ist in Fig. 5 dargestellt. Der $T_m$-Wert beträgt 45,4 °C. Der $T_m$-Wert von AATAT (45 µm) beträgt zum Vergleich 49,4 °C.

## Patentansprüche

1. Verbindung der Formel I

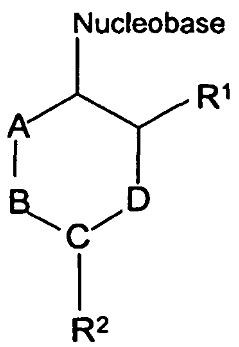

(I)

worin $R^1$ gleich $NR^3R^4$, $OR^3$ oder $SR^3$ ist mit $R^3$ und $R^4$ unabhängig voneinander, gleich oder verschieden, H oder $C_nH_{2n-1}$, wobei n gleich eine ganze Zahl von 1-12 ist;
$R^2$ gleich $C_mH_{2m}$-C(X)-Y mit X gleich =O oder =S ist; Y gleich $OR^3$, $NR^3R^4$ oder $SR^3$ ist, wobei $R^3$ und $R^4$ die oben genannte Bedeutung haben, und m eine ganze Zahl von 1-4, oder
$R^2$ gleich $C_mH2_m$-Z-Y' ist mit Z gleich eine Sulfonyl-, Phosphonyl-, Ether- oder Amin-Gruppe, wobei, wenn Z gleich eine Sulfonyl- oder Phosphonyl-Gruppe, Y' gleich H, $C_nH_{2n+1}$, $OR^3$, $NR^3R^4$ oder $SR^3$ ist, wobei n, $R^3$ und $R^4$ die oben genannte Bedeutung haben, und, wenn Z gleich eine Ether- oder Amin-Gruppe, Y' gleich $C_nH_{2n+1}$, ist;
A, B und D unabhängig voneinander, gleich oder verschieden, $CR^5R^6$ bedeuten mit $R^5$, $R^6$ unabhängig voneinander H oder $C_nH_{2+1}$, wobei n die oben genannte Bedeutung hat; und
C gleich $CR^8$ bedeutet mit $R^8$ unabhängig davon die Bedeutung von $R^5$; und Nucleobase Thymin, Uracil, Adenin, Cytosin, Guanin, Isocytosin, Isoguanin, Xanthin oder Hypoxanthin bedeutet, wobei die Verbindungen der Formel I
1-[2-(tert.-Butoxycarbonyl)-amino-4-(carboxymethyl)cyclohexyl]thymin,
1-[2-(tert-Butoxycarbonyl)-amino-4-(carboxymethyl)cyclohexyl]-3-[(Benzyl oxy)methyl]-thymin und
1-[2-(tert-Butoxycarbonyl)-amino-4-(carboxymethyl)cyclohexyl]-6-N-p-met hoxybenzoyl-adenin,
nicht umfassen.

2. Verbindung nach Anspruch 1, **dadurch gekennzeichnet, daß** $R^1$ gleich $NR^3R^4$ oder $OR^3$ ist und $R^2$ gleich $C_mH_{2m}$-C(X)-Y mit Y gleich $OR^3$ oder $NR^3R^4$ ist.

3. Verbindung nach einem der Ansprüche 1-2, **dadurch gekennzeichnet, daß** $R^1$ gleich $NH_2$ und $R^2$ gleich $CH_2$-COOH ist.

4. Verbindung nach einem der Ansprüche 1-3, **dadurch gekennzeichnet, daß** sie ausgewählt ist aus einer [2-Amino-4-(carboxymethyl)cyclohexyl]-Nucleobase.

5. Verbindung nach einem der Ansprüche 1-4, **dadurch gekennzeichnet, daß** sie ausgewählt ist aus
1-[2-Amino-4-(carboxymethyl)cyclohexyl]-thymin,
1-[2-Amino-4-(carboxymethyl)cyclohexyl]-uracil,
1-[2-Amino-4(carboxymethyl)cyclohexyl]-cytosin,
9-[2-Amino-4-(carboxymethyl)cyclohexyl]-adenin oder
9-[2-Amino-4-(carboxymethyl)cyclohexyl]-guanin.

6. Verbindung nach einem der Ansprüche 1-5, **dadurch gekennzeichnet, daß** die Verbindung enantiomerenrein ist.

7. Verbindung nach einem der Ansprüche 1-6, **dadurch gekennzeichnet, daß** $R^1$ und/oder die Nucleobase mit Schutzgruppen versehen ist.

8. Verbindung nach Anspruch 7, **dadurch gekennzeichnet, daß** die Schutzgruppen ausgewählt sind aus BOC-, BOM-, FMOC-, Ether- oder Acetalschutzgruppen.

9. Verbindung nach einem der Ansprüche 1-8, **dadurch gekennzeichnet, daß** die Nucleobase äquatorial angeordnet ist.

10. Verfahren zur Herstellung einer Verbindung nach einem der Ansprüche 1-9, **dadurch gekennzeichnet, daß**

    a) ein Jodlactam an eine geschützte Nucleobase gekoppelt wird und
    b) das Lactam aus dem vorhergehenden Schritt aktiviert wird, und
    c) anschließend der Lactamring nucleophil geöffnet wird.

11. Verfahren nach Anspruch 10, **dadurch gekennzeichnet, daß** das Jodlactam enantiomerenrein vorliegt.

12. Verfahren nach Anspruch 10 oder 11, **dadurch gekennzeichnet, daß** das Lactam durch Einführen einer Schutzgruppe aktiviert wird.

13. Verfahren nach einem der Ansprüche 10-12, **dadurch gekennzeichnet, daß** der Lactamring gemäß Schritt (c) durch ein Hydroperoxid nucleophil geöffnet wird.

14. Oligomer enthaltend eine Verbindungen gemäß einem der Ansprüche 1-9, oder einer Verbindung ausgewählt aus der Gruppe
1-[2-(tert.-Butoxycarbonyl)amino-4-(carboxymethyl)cyclohexyl]thymin,
1-[2-(tert-Butoxycarbonyl)-amino-4-(carboxymethyl)cyclohexyl]-3-[(Benzyl oxy)methyl]-thymin und
1-[2-(tert-Butoxycarbonyl)-amino-4-(carboxymethyl)cyclohexyl]-6-N-p-met hoxybenzoyl-adenin, die eine 2'-4'-Verknüpfung aufweisen mit Ausnahme von Oligomeren aus der Gruppe CNA(AT), CNA(TAT), CNA(ATAT), CNA(AATAT), wobei die CNA-Bausteine S-Konfiguration haben und die Oligomere über ihr C-terminales Thymin an ein Polyoxyethylen-(POE)-Polystyrol-Copolymer (Tentagel S-HMB-Harz, 0,23 mmol/g) gekoppelt sind, das mit Hydroxybenzol-(HMB)-Linkern derivatisiert ist und wobei die N-terminalen Enden mit einer BOC-Schutzgruppe versehen sein können und wobei die $NH_2$-Gruppe der Adenine durch eine p-Methoxybenzoylgruppe geschützt sind, und mit Ausnahme eines CNA(AATAT)-Oligomers.

15. Oligomer nach Anspruch 14, **dadurch gekennzeichnet, daß** es zusätzlich mindestens einen Linker enthält.

16. Oligomer nach Anspruch 15, **dadurch gekennzeichnet, daß** der Linker ein Lysin-Linker ist.

17. Oligomer nach einem der Ansprüche 15-16, **dadurch gekennzeichnet, daß** es mit einem aktivierten Linker derivatisiert ist.

18. Oligomer nach Anspruch 17, **dadurch gekennzeichnet, daß** der aktivierte Linker Jodacetylsuccinimid und/oder Bis(hydroxysuccinimidyl)glutarat ist.

19. Oligomer nach einem der Ansprüche 14-18, **dadurch gekennzeichnet, daß** das Oligomer phosphatiert ist.

**20.** Oligomer gemäß einem der Ansprüche 14-19, **dadurch gekennzeichnet, daß** es mit einem Biomolekül konjugiert ist.

**21.** Oligomer nach Anspruch 20, **dadurch gekennzeichnet, daß** das Biomolekül ein Peptid, Peptoid, Protein, Zellbestandteil, Fiiamentbestandteil, oder eine Nucleinsäure, und Derivate davon ist.

**22.** Verbindung gemäß einem der Ansprüche 1-9 und/oder einem Oligomer gemäß einem der Ansprüche 14-21, **dadurch gekennzeichnet, daß** mindestens eine dieser Verbindungen und/oder Oligomere auf einem Träger immobilisiert sind.

**23.** Verwendung einer Verbindung gemäß einem der Ansprüche 1-9 und/oder eines Oligomers gemäß einem der Ansprüche 14-21 und/oder eines Trägers gemäß Anspruch 22 in einem Paarungs- und/oder Testsystem.

**Claims**

**1.** Compound of formula I

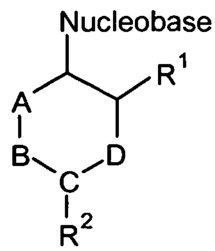

(I)

wherein $R^1$ is equal to $NR^3R^4$, $OR^3$ or $SR^3$ where $R^3$ and $R^4$ independently of one another, identically or differently, are H or $C_nH_{2n+1}$, where n is equal to an integer from 1-12,
$R^2$ is equal to $C_mH_{2m}$-C(X)-Y wherein X is equal to =O or =S; Y is equal to $OR^3$, $NR^3R^4$ or $SR^3$, where $R^3$ and $R^4$ have the abovementioned meaning, and m is an integer from 1-4, or
$R^2$ is equal to $C_mH_{2m}$-Z-Y' wherein Z is equal to a sulphonyl, phosphonyl, ether or amine group, wherein, if Z is equal to a sulphonyl or phosphonyl group, Y' is equal to H, $C_nH_{2n+1}$, $OR^3$, $NR^3R^4$ or $SR^3$, wherein n, $R^3$ and $R^4$ have the abovementioned meaning, and, if Z is equal to an ether or amine group, Y' is equal to $C_nH_{2n+1}$;
A, B and D independently of one another, identically or differently, are $CR^5R^6$ where $R^5$, $R^6$ independently of one another are H or $C_nH_{2n+1}$, where n has the abovementioned meaning; and
C is equal to $CR^8$ where $R^8$ independently thereof denotes the meaning of $R^5$; and
nucleobase means thymine, uracil, adenine, cytosine, guanine, isocytosine, isoguanine, xanthine or hypoxanthine and wherein the compounds of formula I do not comprise
1-[2-(tert-butoxycarbonyl)-amino-4-(carboxymethyl)cyclohexyl]thymine,
1-[2-(tert-butoxycarbonyl)-amino-4-(carboxymethyl)cyclohexyl]-3-[(Benzyloxy)methyl]-thymine and
1-[2-(tert-butoxycarbonyl)-amino-4-(carboxymethyl)cyclohexyl]-6-N-p-methoxybenzoyl-adenine.

**2.** Compound according to Claim 1, **characterized in that** $R^1$ is equal to $NR^3R^4$ or $OR^3$ and $R^2$ is equal to $C_mH_{2m}$-C(X)-Y wherein Y is equal to $OR^3$ or $NR^3R^4$.

**3.** Compound according to any one of Claims 1-2, **characterized in that** $R^1$ is equal to $NH_2$ and $R^2$ is equal to $CH_2$-COOH.

**4.** Compound according to any one of Claims 1-3, **characterized in that** the compound is selected from a [2-amino-4-(carboxymethyl)cyclohexyl]nucleobase.

**5.** Compound according to any one of Claims 1-4, **characterized in that** the compound is selected from
1-[2-amino-4-(carboxymethyl)cyclohexyl]-thymine,
1-[2-amino-4-(carboxymethyl)cyclohexyl]-uracil,
1-[2-amino-4-(carboxymethyl)cyclohexyl]-cytosine,
9-[2-amino-4-(carboxymethyl)cyclohexyl]-adenine or
9-[2-amino-4-(carboxymethyl)cyclohexyl]-guanine.

**6.** Compound according to any one of Claims 1-5, **characterized in that** the compound is enantiomerically pure.

**7.** Compound according to any one of Claims 1-6, **characterized in that** $R^1$ and/or the nucleobase is provided with protective groups.

**8.** Compound according to Claim 7, **characterized in that** the protective groups are selected from the groups BOC, BOM, FMOC, from ether protective groups or acetal protective groups.

**9.** Compound according to any one of Claims 1-8, **characterized in that** the nucleobase is arranged equatorially.

**10.** Process for the preparation of a compound according to any one of Claims 1-9, **characterized in that**

a) an iodolactam is coupled to a protected nucleobase
b) the lactam from the preceding step is activated, and
c) then the lactam ring is nucleophilic opened.

**11.** Process according to Claim 10, **characterized in that** the iodolactam is present in enantiomerically pure form.

**12.** Process according to Claim 10 or 11, characaterized in that the lactam is activated by introduction of a protective group.

**13.** Process according to any one of Claims 10-12, **characterized in that** the lactam ring is nucleophilic opened by a hydroperoxide according to step (c).

**14.** Oligomer comprising a compound according to one of Claims 1-9 or a compound selected from the group
1-[2-(tert-butoxycarbonyl)-amino-4-(carboxymethyl)cyclohexyl]thymine,
1-[2-(tert-butoxycarbonyl)-amino-4-(carboxymethyl)cyclohexyl]-3-[(Benzyloxy)methyl]-thymine and
1-[2-(tert-butoxycarbonyl)-amino-4-(carboxymethyl)cyclohexyl]-6-N-p-methoxybenzoyl-adenine,
connected via its 2'-4' positions,
with the exception of oligomers of the group
CNA(AT), CNA(TAT), CNA(ATAT), CNA(AATAT), wherein the CNA building blocks have S-configuration and the oligomers are coupled through the C-terminal thymin to a polyoxyethylen-(POE)-polystyrol-copolymer (Tentagel S-HMB-resin, 0,23 mmol/g) derivatised with hydroxybenzoyl-(HMB)-linker and wherein a BOC-protective group may be attached to the N-terminal ends and wherein the $NH_2$-groups of the adenines are protected with an p-methoxybenzoyl group, and with the exception of a CNA(AATAT) oligomer.

**15.** Oligomer according to Claim 14, **characterized in that** it additionally contains at least one linker.

**16.** Oligomer according to Claim 15, **characterized in that** the linker is a lysine linker.

**17.** Oligomer according to any one of Claims 14-16, **characterized in that** the oligomer is derivatized with an activated linker.

**18.** Oligomer according to Claim 17, **characterized in that** the activated linker is iodoacetylsuccinimide and/or bis (hydroxysuccinimidyl) glutarate.

**19.** Oligomer according to any one of Claims 14-18, **characterized in that** the oligomer is phosphated.

**20.** Oligomer according to any one of Claims 14-19, **characterized in that** the oligomer is conjugated with a biomolecule.

**21.** Oligomer according to Claim 20, **characterized in that** the biomolecule is a peptide, peptoid, protein, cell constituent, filament constituent, or a nucleic acid, and derivatives thereof.

**22.** Compound according to any one of Claims 1-9 and/or oligomer according to any one of Claims 14-21, **characterized in that** at least one of the compounds and/or oligomers are immobilized on a carrier.

**23.** Use of a compound according to any one of Claims 1-9 and/or of an oligomer according to one of Claims 14-21 and/or of a carrier according to Claim 22 in a pairing and/or test system.

## Revendications

**1.** Composé de formule 1

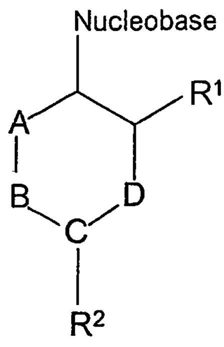

(I)

dans laquelle $R^1$ représente $NR^3R^4$, $OR^3$ ou $SR^3$, $R^3$ et $R^4$ étant indépendamment l'un de l'autre identiques ou différents, H ou $C_nH_{2n+1}$, où n est un nombre entier de 1 à 12;
$R^2$ est $C_mH_2m$-C(X)-Y avec X représentant =O ou =S ; Y représentant $OR^3$, $NR^3R^4$ ou $SR^3$, $R^3$ et $R^4$ ayant la signification donnée précédemment, et m étant un nombre entier entre 1 et 4 ; ou
$R^2$ représente $C_mH_{2m}$-Z-Y', où Z représente un groupe sulfonyle, phosphonyle, éther ou amine, où quand Z représente un groupe sulfonyle ou phosphonyle, Y' représente H, $C_2H_{2n+1}$, $OR^3$, $NR^3R^4$ ou $SR^3$, où n, $R^3$ et $R^4$ ont la signification donnée précédemment, et quand Z est un groupe éther ou amine, Y' représente $C_nH_{2n+1}$;
A, B et D indépendamment les uns des autres sont identiques ou différents et représentent $CR^5R^6$, $R^5$ et $R^6$ représentant indépendamment l'un de l'autre H ou $C_nH_{2n+1}$, où n a la signification donnée précédemment ; et
C représente $CR^8$ avec $R^8$ ayant indépendamment la signification de $R^5$ ; et
la nucléobase représente la thymine, l'uracile, l'adénine, la cytosine, la guanine, l'isocytosine, l'isoguanine, la xanthine ou l'hypoxanthine, les composés de formule 1 ne comprenant pas la 1-[2-(tert.-butoxycarbonyl)-amino-4-(carboxyméthyl)cyclohexyl]thymine, la 1-[2-(tert.butoxycarbonyl)-amino-4-(carboxyméthyl)cyclohexyl]-3-[(benzyloxy)méthyl]-thymine et la 1-[2-(tert.-butoxycarbonyl)-amino-4-(carboxyméthyl)cyclohexyl]-6-N-p-méthoxybenzoyl-adénine.

**2.** Composé selon la revendication 1, **caractérisé en ce que** $R^1$ représente $NR^3R^4$ ou $OR^3$ et $R^2$ représente $C_mH_{2m}$-C(X)-Y, avec Y représentant $OR^3$ ou $NR^3R^4$.

**3.** Composé selon l'une des revendications 1 à 2, **caractérisé en ce que** $R^1$ représente $NH_2$ et $R^2$ représente $CH_2$-COOH.

**4.** Composé selon l'une des revendications 1 à 3, **caractérisé en ce qu'**il est sélectionné parmi une [2-amino-4-(carboxyméthyl)cyclohexyl]-nucléobase.

**5.** Composé selon l'une des revendications 1 à 4, **caractérisé en ce qu'**il est sélectionné parmi
la 1-[2-amino-4-(carboxyméthyl)cyclohexyl]-thymine,
le 1-[2-amino-4-(carboxyméthyl)cyclohexyl]-uracile,
la 1-[2-amino-4-(carboxyméthyl)cyclohexyl]-cytosine,
la 1-[9-amino-4-(carboxyméthyl)cyclohexyl]-adénine ou
la 9-[2-amino-4-(carboxyméthyl)cyclohexyl]-guanine.

**6.** Composé selon l'une des revendications 1 à 5, **caractérisé en ce que** le composé est énantiomériquement pur.

**7.** Composé selon l'une des revendications 1 à 6, **caractérisé en ce que** $R^1$ et/ou la nucléobase est/sont pourvu(s) de groupes protecteurs.

**8.** Composé selon la revendication 7, **caractérisé en ce que** les groupes protecteurs sont sélectionnés parmi des groupes protecteurs BOC-, BOM-, FMOC-, éther ou acétal.

9. Composé selon l'une des revendications 1 à 8, **caractérisé en ce que** la nucléobase a une disposition équatoriale.

10. Procédé de préparation d'un composé selon l'une des revendications 1 à 9, **caractérisé en ce que**

    a) on couple un iodolactame à une nucléobase protégée, et
    b) on active le lactame de l'étape précédente, et
    c) on procède ensuite à l'ouverture nucléophile du cycle lactame.

11. Procédé selon la revendication 10, **caractérisé en ce que** le iodolactame se présente sous une forme énantiomériquement pure.

12. Procédé selon la revendication 10 ou 11, **caractérisé en ce que** le lactame est activé par introduction d'un groupe protecteur.

13. Procédé selon l'une des revendications 10 à 12, **caractérisé en ce que** le cycle lactame subit selon l'étape (c) une ouverture nucléophile par un hydroperoxyde.

14. Oligomère contenant un composé selon l'une des revendications 1 à 9 ou un composé sélectionné dans le groupe de la 1-[2-(tert.-butoxycarbonyl)-amino-4-(carboxyméthyl)cyclohexyl]-thymine, la 1-[2-(tert.-butoxycarbonyl)-amino-4-(carboxyméthyl)cyclohexyl]-3-[(benzyloxy)méthyl]-thymine et la 1-[2-(tert.-butoxycarbonyl)-amino-4-(carboxyméthyl)cyclohexyl]-6-N-p-méthoxybenzoyl-adénine, qui présentent une liaison 2'-4' à l'exception d'oligomères issus du groupe CNA(AT), CNA(TAT), CNA(ATAT), CNA(AATAT), où les éléments CNA ont une configuration S et les oligomères sont couplés par leur thymine C-terminale à un copolymère de polyoxyéthylène-(POE)-polystyrène (résine Tentagel S-HMB, 0,23 mmol/g) qui est dérivé avec des séquences de liaison hydroxybenzène-(HMB) et où les extrémités N-terminales peuvent être munies d'un groupe protecteur BOC et où le groupe $NH_2$ de l'adénine est protégé par un groupe p-méthoxybenzoyle, et à l'exception d'un oligomère CNA (AATAT).

15. Oligomère selon la revendication 14, **caractérisé en ce qu'**il contient en plus au moins une séquence de liaison.

16. Oligomère selon la revendication 15, **caractérisé en ce que** la séquence de liaison est une séquence de liaison lysine.

17. Oligomère selon l'une des revendications 15 à 16, **caractérisé en ce qu'**il est dérivé avec une séquence de liaison activée.

18. Oligomère selon la revendication 17, **caractérisé en ce que** la séquence de liaison activée est un succinimide de iodacétyle et/ou un glutarate de bis(hydroxy)succinimidyle.

19. Oligomère selon l'une des revendications 14 à 18, **caractérisé en ce que** l'oligomère est phosphaté.

20. Oligomère selon l'une des revendications 14 à 19, **caractérisé en ce qu'**il est conjugué à une biomolécule.

21. Oligomère selon la revendication 20, **caractérisé en ce que** la biomolécule est un peptide, un peptoïde, une protéine, un composant cellulaire, un composant de filament, ou un acide nucléique et des dérivés de ceux-ci.

22. Composé selon l'une des revendications 1 à 9 et/ou oligomère selon l'une des revendications 14 à 21, **caractérisé en ce qu'**au moins un de ces composés et/ou oligomères est/sont immobilisé(s) sur un support.

23. Utilisation d'un composé selon l'une des revendications 1 à 9 et/ou d'un oligomère selon l'une des revendications 14 à 21 et/ou d'un support selon la revendication 22 dans un système d'appariement et/ou de test.

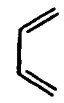

1

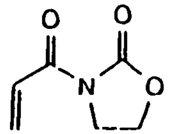

2

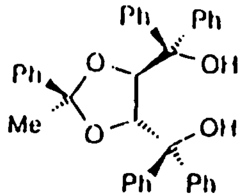

3

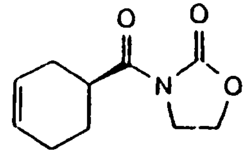

4

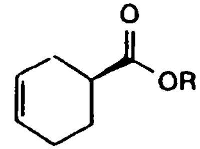

5

b: R = Me

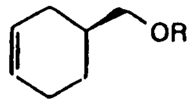

6

a: R = H

b: R = $SO_2Me$

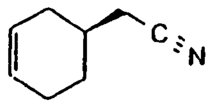

7

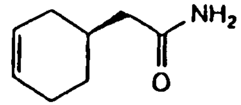

8

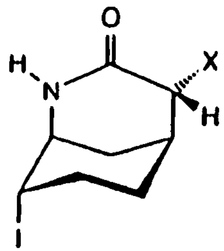

9

X = H

1 + 2 87% 4/*ent*-4 = 96 : 4 93% 5b/*ent*-5b = 95.5 : 4.5 99% 6a/*ent*-6a = 94.5 : 5.5

94% 7/*ent*-7 = 94.5 : 5.5 88% 8/*ent*-8 = 94.5 : 5.5 64% 9 /*ent*-9. > 99 : 1

Fig. 1

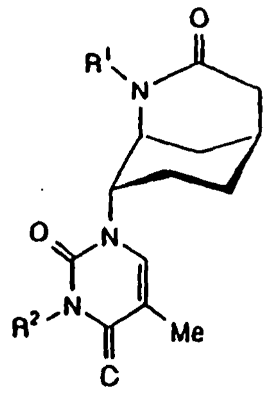

13

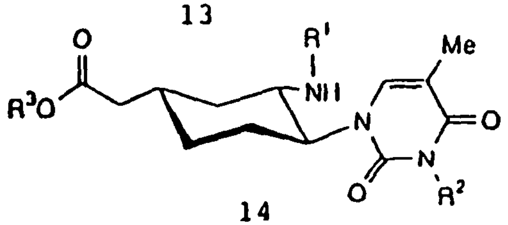

14

13b : $R^1$ = H
$R^2$ = BOM

13c : $R^1$ = BOC
$R^2$ = BOM

14a : $R^1$ = BOC
$R^2$ = BOM
$R^3$ = H

14b : $R^1$ = BOC
$R^2$ = H
$R^3$ = H

9 53% 13b 86% 13c 63% 14a 94% 14b

Fig. 2

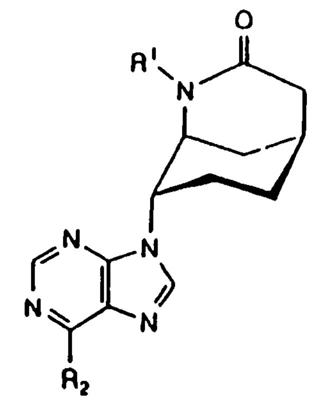

15

**15a** : $R^1$ = H; $R^2$ = HN-CO-Ph
**15b** $R^1$ = H; $R^2$ = $NH_2$
**15c** : $R^1$ = H; $R^2$ = N=C-$(NMe_2)_2$
**15d** : $R^1$ = Boc; $R^2$ = N=C-$(NMe_2)_2$
**15e** : $R^1$ = Boc; $R^2$ = $NH_2$
**15f** : $R^1$ = Boc; $R^2$ = NH-CO-$C_6H_4$-OMe

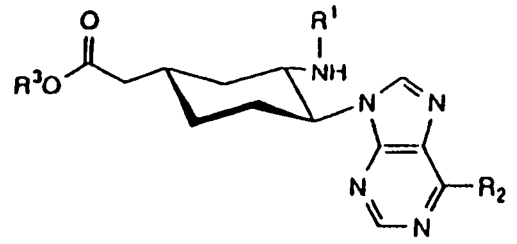

16

$R^1$ = Boc; $R^2$ = NH-CO-$C_6H_4$-OMe; $R^3$ = H

9 73% 15a 97% 15b 53% 15c 78% 15d

86% 15e 88% 15f 46% 16

Fig. 4

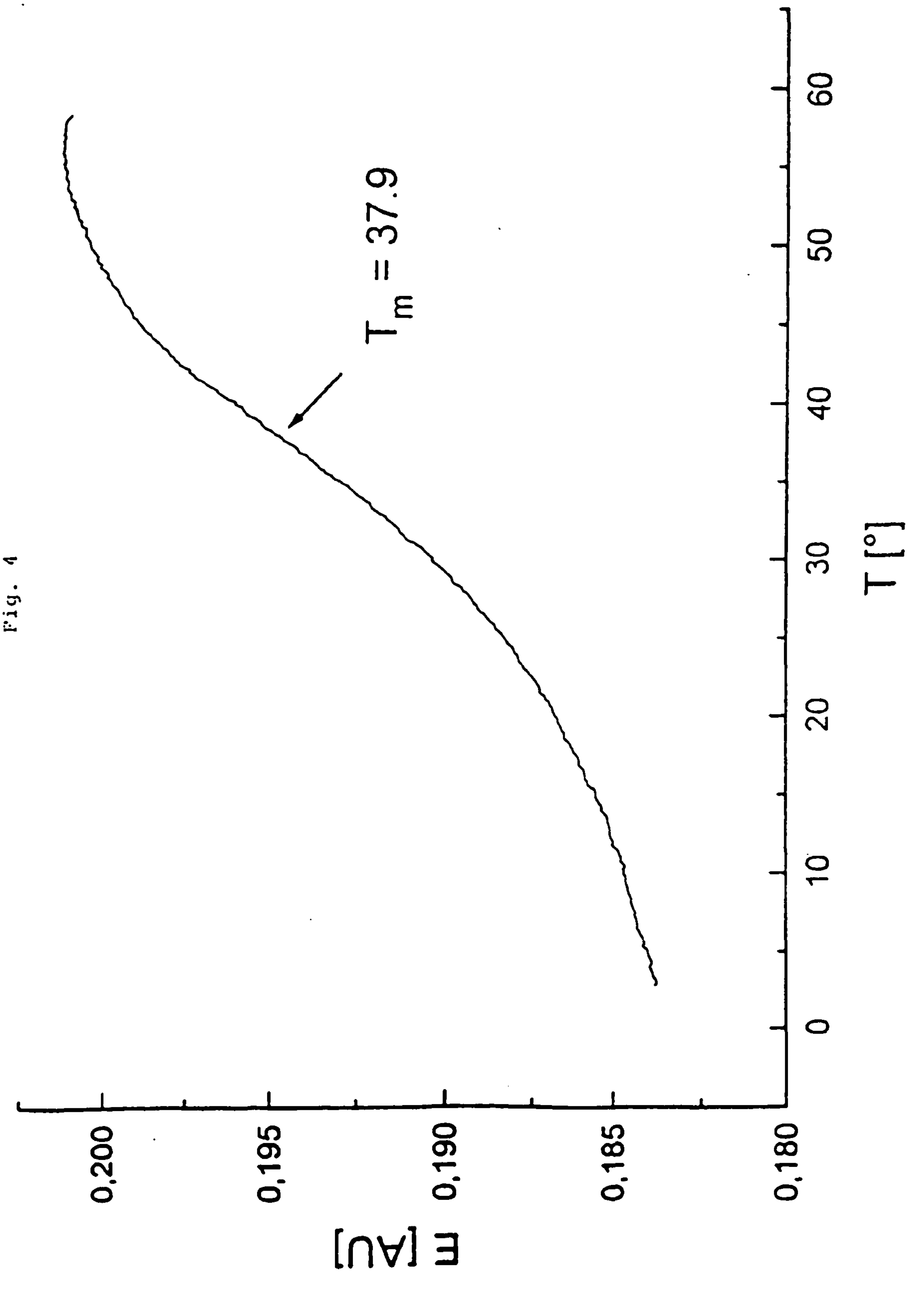

Fig. 5

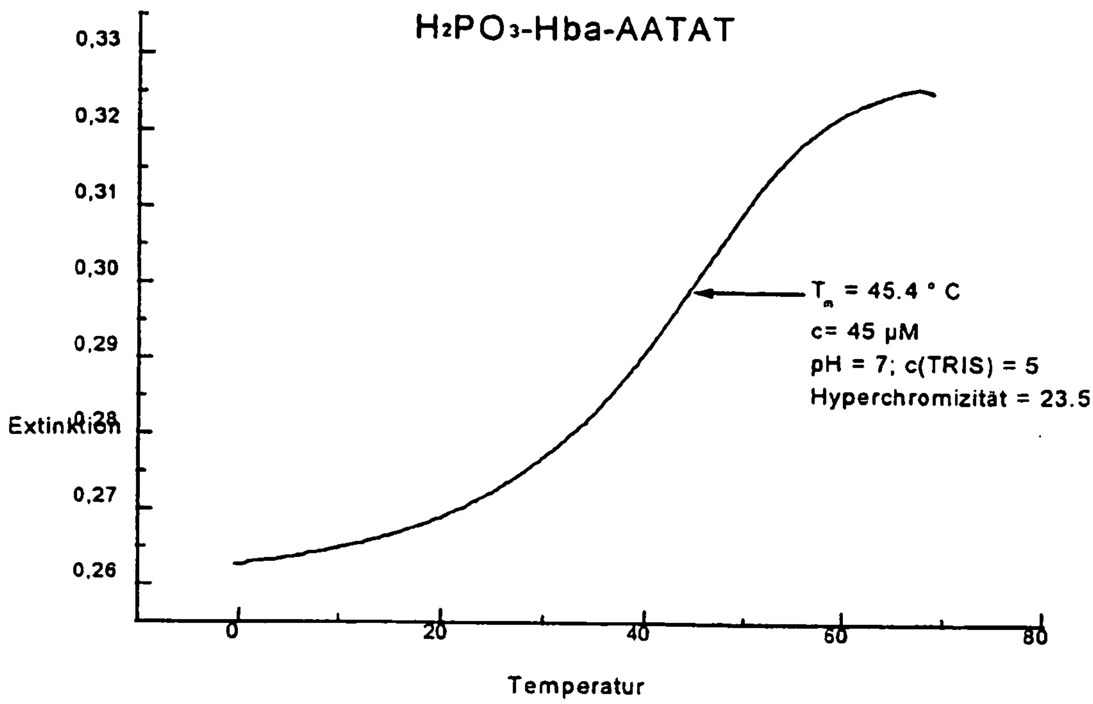
H2PO3-Hba-AATAT
0,33
0,32
0,31
0,30
0,29
0,28
0,27
0,26
Extinktion
0
20
40
60
80
Temperatur
Tm = 45.4 ° C
c= 45 µM
pH = 7; c(TRIS) = 5
Hyperchromizität = 23.5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9613522 A
- WO 9313121 A
- WO 9743232 A
- WO 9613613 A
- WO 9428173 A


### In der Beschreibung aufgeführte Nicht-Patentliteratur


- **R. L. LETSINGER et al.** *Nature,* 1996, vol. 382, 607-9
- **P. G. SCHULTZ et al.** *Nature,* 1996, vol. 382, 609-11
- **B. A. LOMBARDI ; J. W. BRYSON ; W. F. DEGRADO.** *Biomoleküls (Pept. Sci.,* 1997, vol. 40, 495-504
- **SCHNEFFER H.J. et al.** *Journal of Medicinal Chemistry,* Januar 1968, vol. 11 (1), 15-20
- **PEREZ-PEREZ MJ. et al.** *Journal of Organic Chemistry,* Juni 1995, vol. 60 (6), 1531-1531
- **MIKHAILOV S:N. et al.** *Nucleosides & Nucleotides,* 1996, vol. 15 (4), 867-878
- **MARQUEZ V.E. et al.** *Medicinal Research Reviews,* 1986, vol. 6 (1), 1-40
- **BORTHWICK AD. et al.** *Tetrahedron,* 1992, vol. 48 (4), 571-623
- **KAPP S.** *Advances in Heterocyclic Natural Product Synthesis,* 1996, vol. 3, 57-98
- **D. SEEBACH et al.** *J. Org. Chem.,* 1995, vol. 60, 1788
- **S. KNAPP et al.** *Org. Synth.,* 1991, vol. 70, 101
- **R. B. MERRIFIELD.** *J. Amer. Chem. Soc.,* 1963, vol. 85, 2149
- **SKERRA ; PLÜCKTHUN.** *Science,* 1988, vol. 240, 1038
- **BIRD et al.** *Science,* 1988, vol. 242, 423
- **HUSTON et al.** *Proc. Natl. Acad. Sci. U.S.A.,* 1988, vol. 85, 5879
- **BETTER et al.** *Science,* 1988, vol. 240, 1041
- **A. LOMBARDI ; J. W. BRYSON ; W. F. DEGRADO.** *Biomoleküls (Pept. Sci.,* 1997, vol. 40, 495-504